# EUROPEAN PATENT SPECIFICATION

(11) **EP 4 241 740 B1**
(45) Date of publication and mention of the grant of the patent: **15.10.2025**
(21) Application number: 23188300.0
(22) Date of filing: 14.07.2022
(51) Int. Cl.: A61F 2/38, A61F 2/30

(54) **ENDOPROSTHETIC ROTATING HINGE KNEE ASSEMBLIES AND SUBASSEMBLIES**
ENDOPROTHETISCHE DREHBARE GELENKKNIEANORDNUNGEN UND UNTERBAUGRUPPEN
ENSEMBLES ET SOUS-ENSEMBLES DE GENOU ENDOPROTHÉTIQUE À CHARNIÈRE ROTATIVE

(30) Priority: 23.07.2021 US 202163225109 P; 22.04.2022 US 202217660252
(43) Date of publication of application: 13.09.2023
(62) Divisional of application: 22184854.2
(73) Proprietor: MicroPort Orthopedics Holdings Inc., Arlington, TN 38002 (US)
(72) Inventor: BOWMAN, Fred W., Arlington, TN 38002 (US); MOUNTJOY, Kristen F., Allendale, NJ 07401 (US); BROOKS, Michael L., Arlington, TN 38002 (US); CARROLL, Jacob H., Oakland, TN 38060 (US)
(74) Representative: Novagraaf Group

(56) References cited:
- EP-A1- 3 127 510
- WO-A1-2019/210323
- US-A1- 2009 088 860
- US-A1- 2019 336 297

## Description

### BACKGROUND OF THE INVENTION

### 1. Technical Field

The present disclosure relates generally to the field of knee implants, and more particularly to rotating hinge implants for revision and primary knees.

### 2. Related Art

Rotating hinged knee implants are typically used in revision knee surgeries in situations where the natural ligaments and other retaining anatomy of the patient's knee are severely compromised. Non-rotating hinged knee implants were initially used to replace the stabilizing function of a missing posterior cruciate ligament ("PCL"). However, surgeons recognized early on that simple hinge knees were prone to accelerated wear and failure. This problem was addressed by providing a rotating hinge function, such that the tibia rotates around a generally vertical tibial axis relative to the femur during flexion. Several designs have been used for this purpose. See *e.g.,* U.S. Pat. No 6,773,461 and U.S. Pat. No. 10,682,236.

Some drawbacks of conventional rotating hinge knees include: the hinge pin is often inserted from the lateral side, which requires multiple incisions into the leg. Some hinge rotating assemblies, like the one disclosed in U.S. Pat. No 6,773,461 must be assembled intraoperatively. Such designs increase the duration of the procedure. Increased procedure duration likewise increases the risk of infection and other complications attributable to time under anesthesia. Other designs, such as the rotating hinge assembly disclosed in U.S. Pat. No. 10,682,236 and U.S. Pub. No. 2009/088860, require multiple locking mechanisms. Such mechanism also increase installation and overall procedure time. Complicated locking mechanisms can also be difficult to undo in the event that the patient undergoes a future revision surgery.

Furthermore, the devices disclosed in both U.S. Pat. No 6,773,461 and U.S. Pat. No. 10,682,236, and WO Pat. Pub No. 2019/210323 have retaining elements that are inserted and disposed around the tibial rotational axis. In operation, transverse load-bearing elements such as screw threads that are disposed around the tibial rotational axis generally experience significant compressive forces from the femur. This, coupled with torsional forces that the screw threads may experience during normal bending and rotation of the knee joint, may cause these threaded elements (or their associated parts) to fail prematurely. Premature implant failure can result in an otherwise avoidable further revision surgery to fix or replace the implant. Replacing the entire implant often involves cutting away the bone into which the implant was attached. Rotating hinge assemblies are frequently used on patients that already suffer from significant bone degradation. Further revising a failed rotating hinge assembly may not be possible in certain cases if insufficient bone remains.

EP Pat. Pub. No. 3,127, 510 discloses a modular hinge knee prothesis having a hinge pin extending laterally into the prothesis.

US Pat. Pub. No. 2019/0336297 discloses an orthopedic prosthetic system for a constrained knee prothesis of the preamble of claim 1.

### SUMMARY

There is therefore a need for an improved rotating hinge knee having the properties, characteristics and functionality described herein.

The problem of lengthy and cumbersome surgical installation procedure and the problems of rotating hinge implants failing prematurely due to mechanical wear of internal tibial aligned implant fastening elements can be mitigated by an exemplary knee joint endoprosthetic assembly comprising: a rotating hinge subassembly having a femur box that can hingedly articulate around a tibial yoke (*e.g.*, via a transverse hinge pin), wherein the femur box is configured to be mechanically engaged to a femoral component via a femur fastener, the femur fastener being non-axially aligned with a tibial axis of rotation when the knee is in flexion or extension, and wherein the transverse hinge pin of the rotating hinge subassembly does not mechanically engage the femoral component in an installed configuration.

The claimed invention is defined in the appended claims. It is contemplated that certain exemplary embodiments disclosed herein may allow for implantation of a rotating hinge knee through a single incision.

It is further contemplated that certain exemplary embodiments in accordance with this disclosure may provide a rotating hinge knee having a preassembled rotating hinge subassembly that is not preassembled with the femoral component or the tibial component of the endoprosthetic implant.

It is still further contemplated that certain exemplary embodiments in accordance with the present disclosure may provide a rotating hinge knee having one femur fastener for securing the rotating hinge subassembly to the femoral component of the endoprosthesis, wherein the femur fastener engages the femoral component in a parasagittal plane.

It is yet still further contemplated that certain exemplary embodiments in accordance with this disclosure may disallow the need for excessive dislocation of the distal femur relative to the proximal tibia during endoprosthetic implant installation and assembly, which may minimize resections of surrounding soft tissue and thereby contribute to faster patient recovery.

Still further exemplary embodiments disclosed herein may provide a rotating hinge knee having an extension stop for interaction with the femoral component.

The foregoing objectives are achieved by providing a rotating hinge knee implant assembly having the features described herein.

### BRIEF DESCRIPTION OF THE DRAWINGS

The foregoing will be apparent from the following more particular description of exemplary embodiments of the disclosure, as illustrated in the accompanying drawings. The drawings are not necessarily to scale, with emphasis instead being placed upon illustrating the disclosed embodiments.
**FIG. 1** is front view of an exemplary embodiment of an endoprosthetic rotating hinge knee implant assembly in an assembled state (*i.e*., an installed configuration).
**FIG. 2A** is an anterior perspective view of one exemplary embodiment of a femoral component and a tibial component of an endoprosthetic rotating hinge knee implant assembly.
**FIG. 2B** shows an anterior perspective view of one exemplary embodiment of a rotating hinge subassembly for use in an endoprosthetic rotating hinge knee implant assembly.
**FIG. 2C** shows an anterior perspective view of one exemplary embodiment of a rotating hinge knee implant assembly showing insertion of a rotating hinge subassembly into the tibial component.
**FIG. 2D** shows an anterior perspective view of one exemplary embodiment of an endoprosthetic rotating hinge knee implant assembly showing attachment of a rotating hinge subassembly to the femoral component.
**FIG. 3A** shows a cross-sectional lateral view of one exemplary embodiment of an endoprosthetic rotating hinge knee implant assembly in extension; the cross section is taken from a parasagittal plane that bisects the exemplary endoprosthetic rotating hinge implant assembly.
**FIG. 3B** shows a cross-sectional lateral view of one exemplary embodiment of an endoprosthetic rotating hinge knee implant assembly in flexion; the cross section is taken from a parasagittal plane that bisects the exemplary endoprosthetic rotating hinge implant assembly.
**FIG. 4** shows an exploded view of one embodiment of an exemplary endoprosthetic rotating hinge knee implant assembly and an exploded view of an exemplary rotating hinge knee subassembly.
**FIG. 5** shows a perspective view of one embodiment of a femur box for use in an exemplary rotating hinge subassembly.
**FIG. 6** shows a perspective view of one embodiment of a femur box bearing member for use with an exemplary endoprosthetic rotating hinge knee implant assembly.
**FIG. 7A** shows a lateral view of one embodiment of an exemplary tibial yoke for use in an endoprosthetic rotating hinge subassembly.
**FIG. 7B** depicts an anterior view of the exemplary embodiment of the tibial yoke depicted in **FIG. 7A****.**
**FIG. 7C** depicts a top down view of the exemplary embodiment of the tibial yoke depicted in **FIGs. 7A** and **7B****.**
**FIG. 7D** is a bottom-up view of the exemplary embodiment of the tibial yoke depicted in **FIGs. 7A, 7B,** and **7C****.**
**FIG. 8A** shows a top-side perspective view of one embodiment of a modular extension stop for use in a rotating hinge subassembly.
**FIG. 8B** shows a lateral view of one embodiment of a modular extension stop for use in a rotating hinge subassembly.
**FIG. 8C** shows an anterior view of one embodiment of a modular extension stop for use in a rotating hinge subassembly.
**FIG. 9** shows a perspective leading end to trailing end view of one embodiment of a femur fastener for use with a rotating hinge knee implant assembly.
**FIG. 10A** shows a top-side perspective view of another embodiment of an extension stop for use with a rotating hinge knee implant assembly.
**FIG. 10B** shows a lateral view of another embodiment of a modular extension stop for use with a rotating hinge knee implant assembly.
**FIG. 10C** shows an anterior view of another embodiment of a modular extension stop for use with a rotating hinge knee implant assembly.
**FIG. 11A** is a perspective view of an exemplary endoprosthetic rotating hinge knee implant assembly showing the rotating hinge subassembly prior to being affixed to the femoral component via a femur fastener.
**FIG. 11B** is a perspective view of an exemplary endoprosthetic rotating hinge knee implant assembly showing the rotating hinge subassembly in an engaged position.
**FIG. 12** is a cross-sectional lateral view of exemplary embodiment of a rotating hinge knee implant assembly in extension showing an alternative modular extension stop; the cross section is taken from a parasagittal plane that bisects the exemplary endoprosthetic rotating hinge implant assembly.
**FIG. 13** is a schematic perspective view of anatomical planes relative to a person.

### DETAILED DESCRIPTION OF THE PREFERRED EMBODIMENTS

The following detailed description of the preferred embodiments is presented only for illustrative and descriptive purposes. The embodiments were selected and described to best explain the principles of the invention and its practical application. One of ordinary skill in the art will recognize that many variations can be made to the invention disclosed in this specification without departing from the scope of the appended claims.

Similar reference characters indicate corresponding parts throughout the several views unless otherwise stated. Although the drawings represent embodiments of various features and components according to the present disclosure, the drawings are not necessarily to scale and certain features may be exaggerated to better illustrate embodiments of the present disclosure, and such exemplifications are not to be construed as limiting the scope of the present disclosure.

Except as otherwise expressly stated herein, the following rules of interpretation apply to this specification: (a) all words used herein shall be construed to be of such gender or number (singular or plural) as such circumstances require; (b) the singular terms "a," "an," and "the," as used in the specification and the appended claims include plural references unless the context clearly dictates otherwise; (c) the antecedent term "about" applied to a recited range or value denotes an approximation with the deviation in the range or values known or expected in the art from the measurements; (d) the words, "herein," "hereby," "hereto," "hereinbefore," and "hereinafter," and words of similar import, refer to this specification in its entirety and not to any particular paragraph, claim, or other subdivision, unless otherwise specified; (e) descriptive headings are for convenience only and shall not control or affect the meaning of construction of part of the specification; and (f) "or" and "any" are not exclusive and "include" and "including" are not limiting. Further, the terms, "comprising," "having," "including," and "containing" are to be construed as open-ended terms (*i.e.*, meaning "including but not limited to").

References in the specification to "one embodiment," "an embodiment," "an exemplary embodiment," *etc.*, indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments, whether explicitly described.

Recitation of ranges of values herein are merely intended to serve as a shorthand method of referring individually to each separate value falling within the range of any sub-ranges there between, unless otherwise clearly indicated herein. Each separate value within a recited range is incorporated into the specification or claims as if each separate value were individually recited herein. Where a specific range of values is provided, it is understood that each intervening value, to the tenth or less of the unit of the lower limit between the upper and lower limit of that range and any other stated or intervening value in that stated range of sub range thereof, is included herein unless the context clearly dictates otherwise. All subranges are also included. The upper and lower limits of these smaller ranges are also included therein, subject to any specifically and expressly excluded limit in the stated range.

It should be noted that some of the terms used herein are relative terms. For example, the terms, "upper" and, "lower" are relative to each other in location, *i.e.,* an upper component is located at a higher elevation than a lower component in each orientation, but these terms can change if the orientation is flipped. The terms, "inlet" and "outlet" are relative to the fluid flowing through them with respect to a given structure, *e.g.,* a fluid flows through the inlet into the structure and then flows through the outlet out of the structure. The terms, "upstream" and "downstream" are relative to the direction in which a fluid flows through various components prior to flowing through the downstream component.

The terms, "horizontal" and "vertical" are used to indicate direction relative to an absolute reference, *i.e.,* ground level. However, these terms should not be construed to require structure to be absolutely parallel or absolutely perpendicular to each other. For example, a first vertical structure and a second vertical structure are not necessarily parallel to each other. The terms, "top" and "bottom" or "base" are used to refer to locations or surfaces where the top is always higher than the bottom or base relative to an absolute reference, *i.e.,* the surface of the Earth. The terms, "upwards" and "downwards" are also relative to an absolute reference; an upwards flow is always against the gravity of the Earth.

Throughout this disclosure, various positional terms, such as "distal," "proximal," "medial," "lateral," "anterior," and "posterior," will be used in the customary manner when referring to the human anatomy. More specifically, "distal" refers to the area away from the point of attachment to the body, while "proximal" refers to the area near the point of attachment to the body. For example, the distal femur refers to the portion of the femur near the tibia, whereas the proximal femur refers to the portion of the femur near the hip. The terms, "medial" and "lateral" are also essentially opposites. "Medial" refers to something that is disposed closer to the middle of the body. "Lateral" means that something is disposed closer to the right side or the left side of the body than to the middle of the body. Regarding, "anterior" and "posterior," "anterior" refers to something disposed closer to the front of the body, whereas "posterior" refers to something disposed closer to the rear of the body."

"Varus" and "valgus" are broad terms and include without limitation, rotational movement in a medial and/or lateral direction relative to the knee joint.

The phrase, "mechanical axis of the femur" refers to an imaginary line drawn from the center of the femoral head to the center of the distal femur at the knee.

The phrase, "mechanical axis of the tibia" refers to an imaginary line drawn from the center of the proximal tibia to the center of the distal tibia, which is just above the ankle.

The term, "anatomic axis" refers to an imaginary line drawn lengthwise down the middle of femoral shaft or tibial shaft, depending upon use.

Described herein are hinge knee assemblies, systems, and methods that may be configured to be implanted in a matter similar to currently available primary and revision surgical techniques.

During primary or revision knee surgeries, the surgeon generally makes a vertical midline incision on the anterior side of the operative knee. The incision is generally made with the knee in flexion at or below the tibial tuberosity and may extend several centimeters above the patella.

In a primary TKA, the surgeon continues to incise the fatty tissue to expose the anterior aspect of the joint capsule. The surgeon may then perform a medial parapatellar arthrotomy to pierce the joint capsule and resect the medial patellar retinaculum. A retractor is then commonly used to move the patella generally laterally to expose the distal condyles of the femur and the cartilaginous meniscus resting on the proximal tibial plateau. The surgeon then removes the meniscus and uses instrumentation to measure and resect the distal femur and proximal tibia to accommodate trial implants. Trial implants are test endoprostheses that generally have the same functional dimensions of the actual endoprostheses, but trial implants are designed to be temporarily installed and removed for the purposes of evaluating the fit of the actual endoprostheses and for the purposes of evaluating the knee joint's kinematics. The surgeon removes the trial implants and installs the actual implants once the surgeon is satisfied with the trial implant's sizing and the knee joint's kinematics.

In a revision procedure, after the surgeon has performed the initial incision, the surgeon may release scar tissue around the patellar tendon. The surgeon then moves (*i.e.,* preforms a subluxation of) the patella or patellar implant generally laterally to expose the prior-installed implant, which usually has a femoral component installed on the distal femur, a tibial component installed on the proximal tibia, and a meniscal insert disposed between the femoral component and the tibial component. The surgeon then removes the prior-installed implant.

It will be appreciated that the type of prior-installed implant can vary from case to case. Prior-installed implants can include static spacers that have been inserted into aligned intramedullary bores in the distal femur and proximal tibia to immobilize the knee joint, or complex implants that have been used to reconstruct portions of the knee joint that had undergone trauma. However, common prior-installed implants include implants installed during a primary TKA, or prior revision implants. A variety of factors influence the decision to replace a prior-installed implant with a rotating hinge knee implant, but common factors include excessive wear or inoperability, the presence of trauma, bone degenerative disease progression, the integrity of the underlying bone, and severe varus/valgus deformities. Common bone degenerative diseases include rheumatoid arthritis and arthrosis.

Prior installed implants may have been bonded to bone in a variety of ways. Press-fit implants typically have a porous roughened surface on the connective side of the femoral and tibial components. The porous surface permits regrowth of the bone into these pours over time. Another very common bonding technique involves the use of an antibiotic infused grout commonly known as "bone cement" by people in the orthopedic industry. It will be appreciated that "bone cement" is a term of art even though bone cements themselves generally do not have adhesive properties. Bone cements generally rely on a close mechanical interlock between the irregular surface of the bone and the surface of the connective side of the endoprosthesis. Common bone cements include polymethyl methacrylate ("PMMA"), calcium phosphate cements ("CPCs"), and glass polyalkenoate isomer cements ("GPICs").

Removal of the prior-installed implants generally involves cutting away the bone underlying the bone cement - or in the case of press-fit implants, the bone underlying the press-fit implant. This resection exposes fresh bone capable of receiving a revision press-fit or bone cement bondable implant. Removing bone to remove the prior-installed implant would move the joint line if the revision implant was not sized to replace the newly resected bone.

This tibial resection is usually coplanar with a transverse body plane that is perpendicular to the anatomic axis of the tibia. Once resected, the resected area of the tibia can be known as the "tibial plateau." After the prior-installed implant has been removed, a series of differently sized intramedullary reamers may then be used to prepare or extend an intramedullary bore in both the tibia and the femur to seat the tibial and femoral components of the revision implant respectively. After reaming, the surgeon may use instrumentation to further measure and resect the proximal tibial plateau. Next, the surgeon may place a trial tibial component on the resected proximal tibial plateau. The surgeon generally uses different instrumentation to measure and resect the distal femoral condyles for the purpose of installing a trial femoral component. If the trial components are not seated appropriately, the surgeon may use further instrumentation to measure and resect the femoral condyles and/or the tibial plateau until the desired seating is achieved.

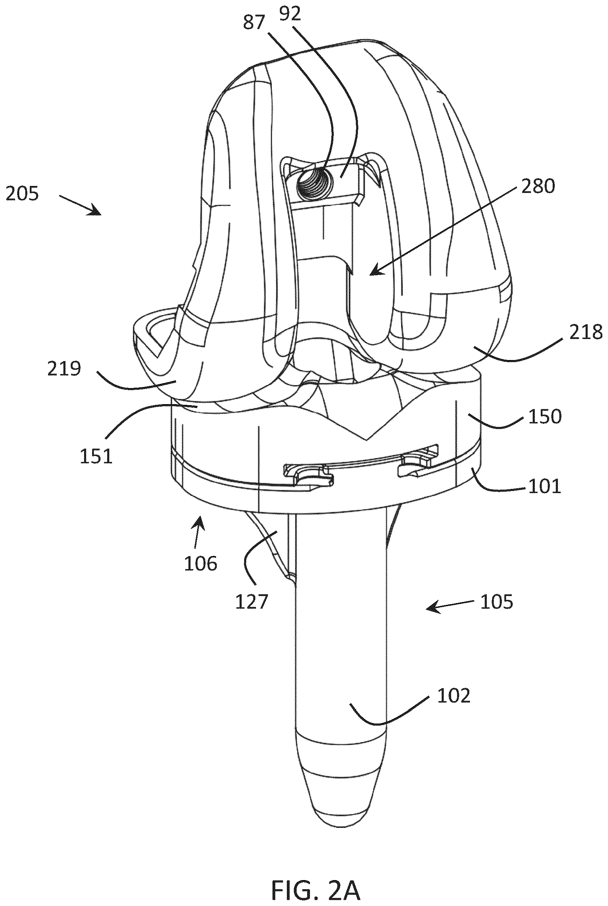

Rotating hinge knees typically have a femur box cavity (see **280,** **FIG. 2A**) in the femoral component (see **205,** **FIG. 2A**) of the endoprosthetic implant. Further resection of the lateral side of the medial condyle and the medial side of the lateral condyle is generally required to accommodate the femur box cavity. Stated differently, the surgeon makes a box-shaped cut out in the middle of the distal femur to accommodate the femur box cavity of the femoral component of the implant. Failure to properly resect the distal femur could result in intercondylar fractures.

The surgeon then generally inserts a trial meniscal insert between the trial tibial tray and the trial femoral component to test the knee's flexion and extension, general stability, and patellar tracking on the trial implants. Metallic blocks called "augments" can be attached to the tibial or femoral components to replace missing or compromised bone. Once satisfied with the trial and movement characteristics, the surgeon can use new uncured antibiotic infused bone cement to permanently affix the actual tibial and femoral components of the endoprosthetic implant or use a press-fit implant and avoid use of bone cement if desired.

Other rotating hinge knee systems differ from the present disclosure in that many other rotating hinge knee systems require intraoperative lateral assembly of the hinge component. This interoperative assembly requires additional resection of the lateral side of one or more femoral condyles to provide access to a hinge component (which is commonly a pin). Some designs require additional posterior resection of the condyles. Additional resections prolong the procedure and reduce the amount of remaining bone available to future revisions. It will also be appreciated that additional but unnecessary lateral and/or posterior condylar resections can prolong the patient's recovery time, introduce new areas that may become infected, and introduce an area of structural weakness - especially in cases with existing bone degradation. Any area of structural weakness increases the risk of catastrophic implant failure during normal use.

Interoperative assembly of a rotating hinge knee also prolongs the procedure and increases the risk of infection and other complications borne from increased time under anesthesia.

Some other rotating hinge knees use retaining elements having screw threads configured to be inserted and disposed around the tibial rotational axis. Without being bound by theory, it is contemplated that in operation, transverse load-bearing elements that are disposed around the tibial rotational axis generally experience significant compressive forces from the femur. This, coupled with torsional forces that the screw threads may experience during normal bending and rotation of the knee joint, may cause parts with these threaded elements to fail prematurely, thereby necessitating further revision surgeries to replace either the worn part or the entire implant.

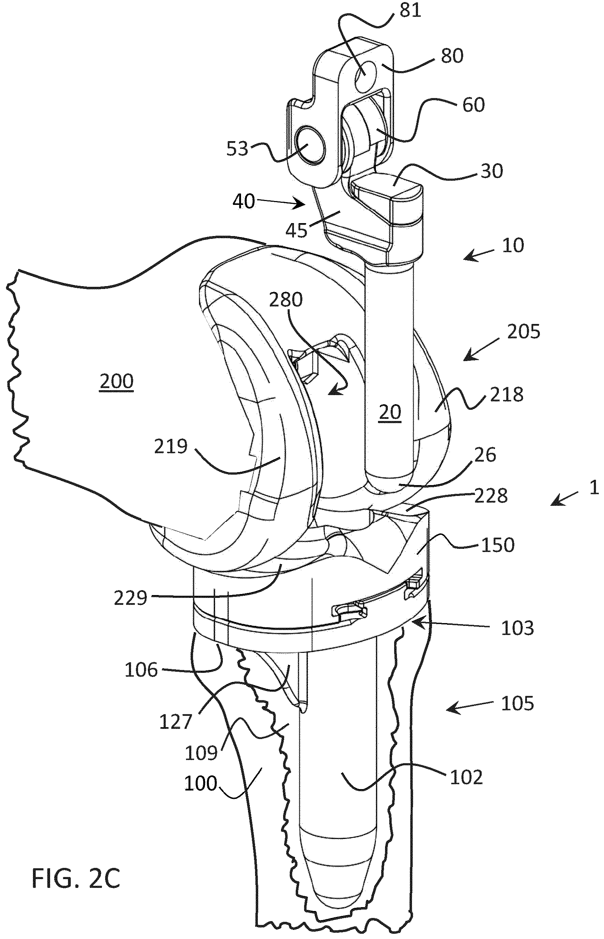

Further, other rotating hinge knees are not configured to restrict five of the six degrees of freedom of the femoral component and the tibial component during installation. It is contemplated that the exemplary embodiments disclosed herein can further facilitate the installation process by permitting the surgeon to align the femoral component with the rotating hinge subassembly (see **10,** **FIG. 2C**) without having to focus on the potential rotation of the femur or tibia during the alignment process.

To reduce installation time compared to existing rotating hinge knees, exemplary embodiments of the rotating hinge subassembly **10** described herein (see *e.g.,* **FIG. 2C**) can come preassembled in a self-contained module that is configured to be mechanically affixed to the femoral component using a single femur fastener **210.**

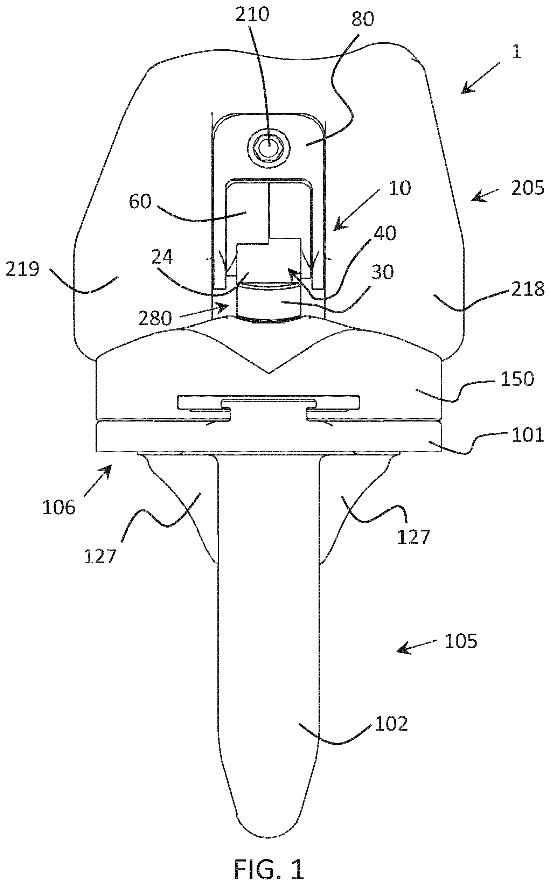

As shown in the assembled front view of **FIG. 1****,** a rotating hinge knee endoprosthetic implant assembly **1** in accordance with this disclosure includes generally: a femoral component **205,** a tibial component **105**, a meniscal insert **150** disposed between the femoral component **205** and the tibial component **105**, and a rotating hinge subassembly **10.** In **FIG. 1****,** many of the rotating hinge subassembly components are obstructed by the femoral component **205** and the tibial component **105**, but some components are visible, such as a femur box **80** disposed in the femur box cavity **280** (**FIG. 2A**) of the femoral component **205**, and a head **24** of the tibial yoke **40.** Bearings **60** are disposed between the head **24** and the femur box **80** to facilitate the hinge movement of the rotating hinge knee endoprosthetic implant assembly **1.** A femur fastener **210** extends through a femur box securing bore **81** in the femur box **80** and a femur securing bore **87** (**FIG. 2A**) in the femoral component **205** to fixedly engage the rotating hinge subassembly **10** to the femoral component **205.** In the depicted embodiment, the femur fastener **210** is a tapered screw and the femur box securing bore **81** and the femur securing bore **87** are threaded to engage the corresponding threads of the tapered screw. In other exemplary embodiments, either the femur securing bore **87,** the femur box securing bore **81,** or both the femur securing bore **87** and the femur box securing bore **81** need not be threaded. An extension stop **30** can be provided to prevent overextension of the rotating hinge knee endoprosthetic implant assembly **1.** In certain exemplary embodiments, the extension stop **30** can be a modular extension stop **30.** That is, a surgeon can select and install one of a variety of available extension stops based on the patient's specific anatomy.

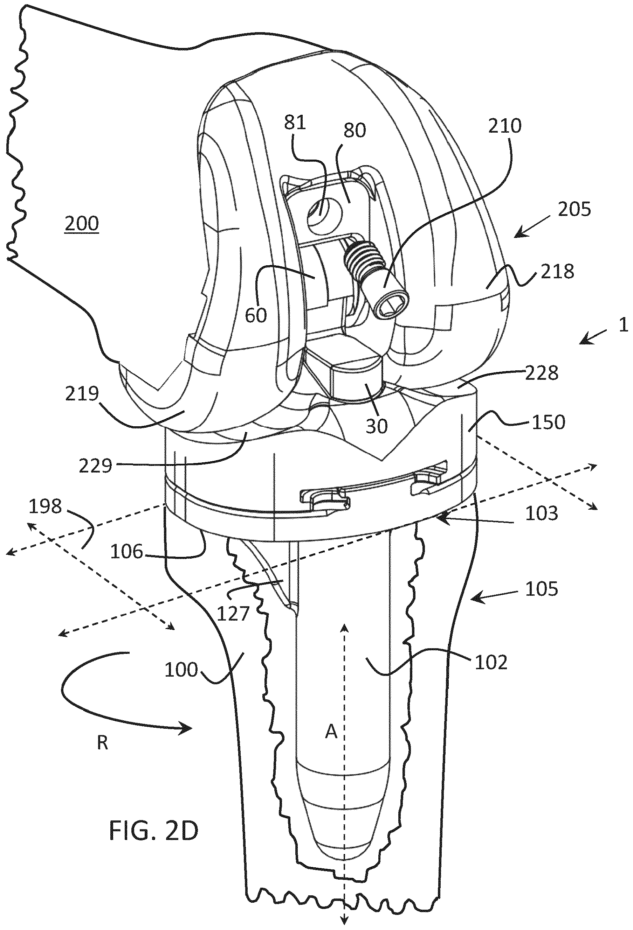

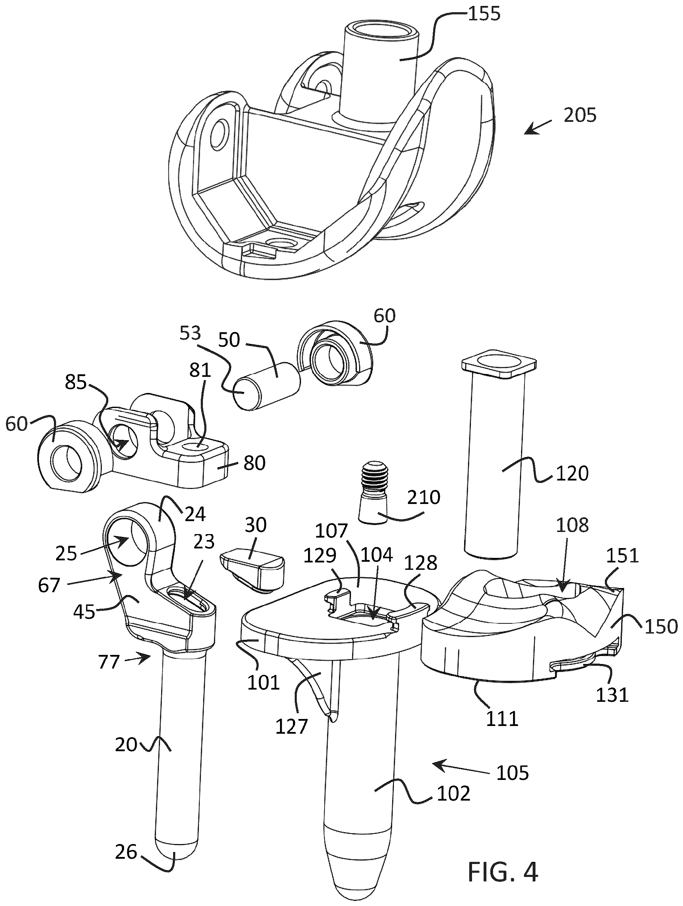

The femoral component **205** comprises a medial implant condyle **218** that is distally disposed from a lateral implant condyle **219.** **FIG. 1** depicts the respective implant condyles **218, 219** resting upon an articular surface **151** (**FIG. 2A**) of a meniscal insert **150.** The meniscal insert **150** is typically made from medical grade polyethylene (*e.g.,* ultra-high molecular weight polyethylene ("UHMWPE")) or other suitable clinically tested biocompatible material. A base portion **101** of the tibial component **105** supports the meniscal insert **150.** The base portion **101** is configured to rest on a patient's resected tibial plateau (**103,** **FIG 2C**). The tibial component **105** can be made from biocompatible material, such as a cobalt-chromium molybdenum alloy, titanium alloy, or other clinically proven high-strength biocompatible material. A tibial stem **102** depends downwardly from the distal side **106** of the tibial base portion **101.** The tibial stem **102** is configured to be inserted into an intramedullary bore (see the depicted cutaway **109**) of the tibia **100.** Keels **127** facilitate installation and securing of the tibial component **105** into an intramedullary bore (see **109**) in the tibia **100** (see **FIG. 2C**). The depicted cutaway **109** is provided to illustrate how the tibial component **105** can be seated in and on the proximal tibia **100** when installed. In practice, the depicted cutaway **109** should generally not exist. It will be appreciated that other tibial components **105** that are compatible with the exemplary assemblies described herein may lack keels **127.** In certain exemplary embodiments, the tibial stem **102** can be configured modularly to have extensions of different lengths to accommodate intramedullary bores of different lengths, angles, or offsets relative to the transverse plane **198** (**FIG. 2D**). In still further exemplary embodiments, the keels **127** can be modular components configured to fixedly engage the tibial component **105.** As better seen in **FIG. 4****,** the tibial stem **102** is substantially hollow on at least an upper/proximal end thereof and has an axial bore **104** configured for the receipt of a longitudinal tibial axial post **20** of a rotating hinge subassembly **10,** as further described below.

Desirably, a sleeve **120** is inserted into the axial bore **104** prior to insertion of the tibial axial post **20.** The sleeve **120** prevents the tibial axial post **20,** which is typically made from a biocompatible metal alloy, from rubbing against the inside of the tibial stem **102,** which is also typically made from a biocompatible metal alloy. Without the sleeve **120,** it is contemplated that the repeated friction of the tibial axial post **20** moving against the inside of the tibial stem **102** could create metal shavings that could undermine the effectiveness and integrity of the endoprosthesis. It is contemplated that the sleeve **120** can be made from UHMWPE, polyether ether ketone ("PEEK"), or other clinically proven biocompatible polymer. In other exemplary embodiments, the sleeve **120** can be made from ceramic materials, including but not limited to zirconia toughened alumina ("ZTA") ceramics. It still other exemplary embodiments, the sleeve **120** can be manufactured from cobalt chromium molybdenum alloys, or titanium allows and coated in zirconium oxide or niobium nitride to further reduce coefficients of friction between the articulating components and to enhance durability. In this manner, the sleeve **120** effectively creates a barrier between the inner wall of the tibial stem **102** and the outer wall of the tibial axial post **20.**

**FIGs. 2A - 2D** provide a series of perspective views showing primary steps of the implantation of the rotating hinge knee endoprosthetic implant assembly **1.**

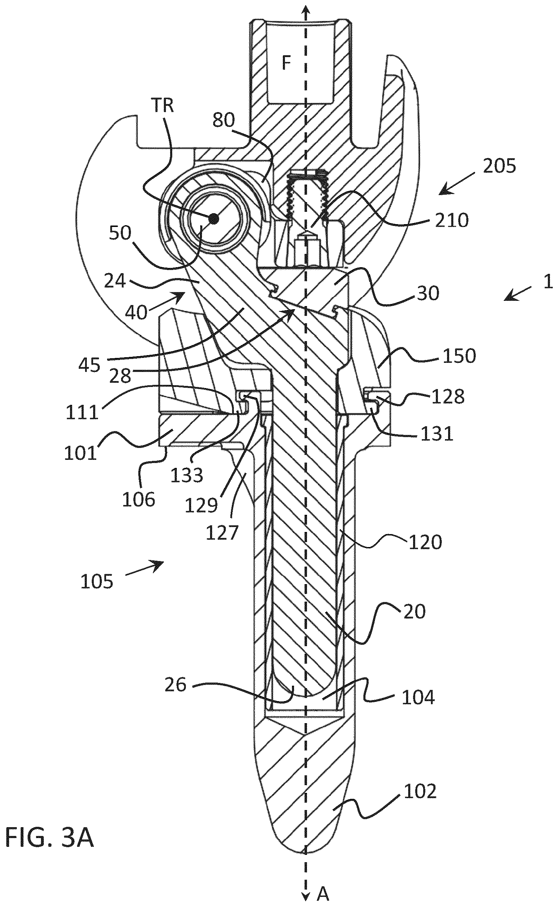

**FIG. 2A** depicts the femoral component **205** oriented in flexion relative to the tibial component **105.** A large femur box cavity **280** is disposed between the medial implant condyle **218** and the adjacent lateral implant condyle **219.** The femur box cavity **280** is configured to receive a femur box **80,** as described below. In the depicted embodiment, the femoral component **205** comprises a femur securing bore **87** that is exposed to the femur box cavity **280.** The femur securing bore **87** is secured in a transverse surface **92** that extends between the inner surfaces of the medial implant condyle **218** and the lateral implant condyle **219.** A meniscal insert **150** is disposed on the base **101** of the tibial component **105** in a rotating or mobile bearing arrangement (see **FIG. 3A** for a discussion of an exemplary rotating bearing arrangement). Various configurations can be used for the connection between the meniscal insert **150** and the tibial base portion **101.**

The femoral component **205** can be made from biocompatible material, such as a cobalt-chromium molybdenum alloy, titanium alloy, or other suitable high-strength biocompatible material. The articular surfaces (*i.e.*, medial implant condyle **218** and a lateral implant condyle **219**) can optimally be coated with a durable biocompatible material that has a low coefficient of friction to provide smooth articular bearing surfaces. Examples of such a coating include zirconium oxide or niobium nitride.

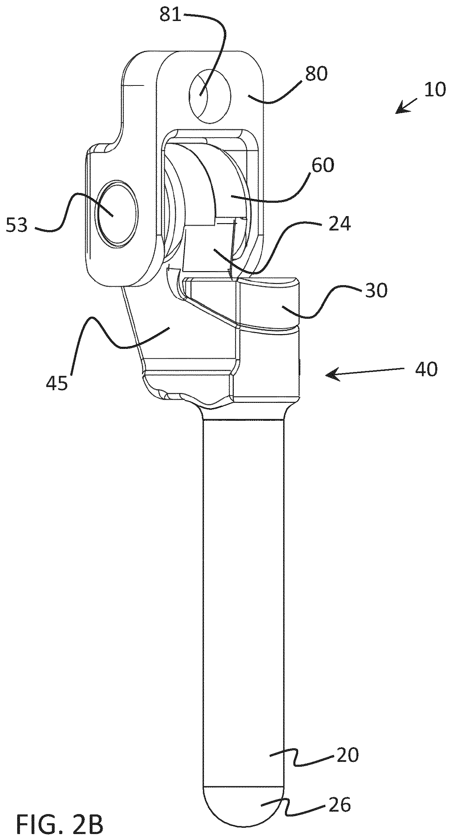

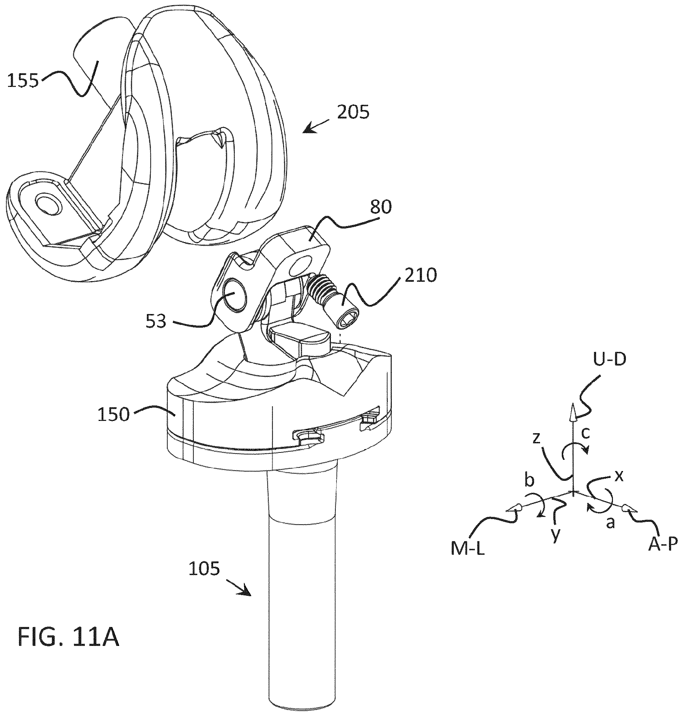

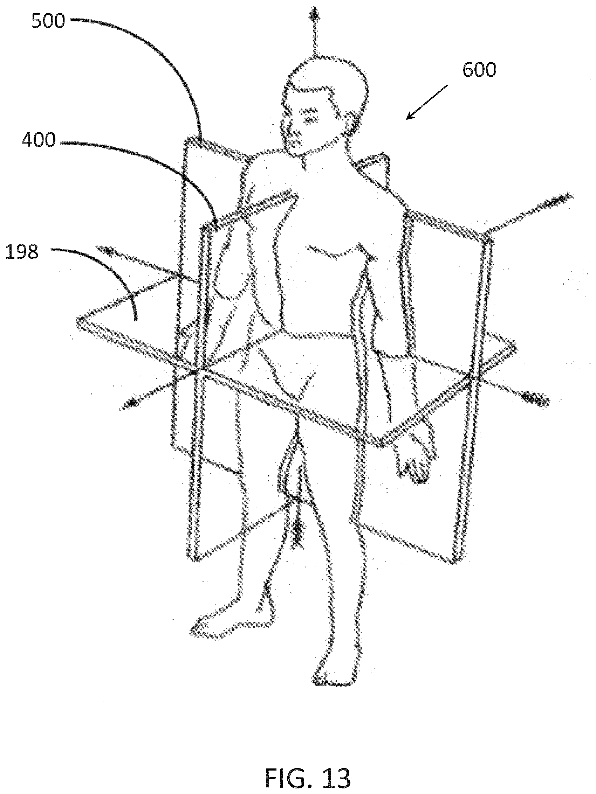

**FIG. 2B** provides a perspective view of a rotating hinge subassembly **10** in a disengaged position, wherein the first joint element (*e.g.,* the femur box **80**) is fully separated from the femoral component **205.** One of the advantages of exemplary embodiments of the present disclosure is that the rotating hinge subassembly **10** can be completely assembled prior to insertion into the patient's knee, thus facilitating the implantation procedure and contributing to reduced operating time. A preassembled rotating hinge subassembly **10** comprises a yoke **40** and a femur box **80** that hingedly articulates around the head **24** of the yoke **40.** The depicted femur box **80** comprises a femur box securing bore **81.** It will be appreciated that a femur box securing bore **81** is an example of a femoral fastening mechanism that can be used to selectively engage the femur box **80** to the femur box cavity **280** of the femoral component. In other exemplary embodiments, it will be appreciated that the femoral fastening mechanism may comprise a projection, a recession, a receiver, multiple projections, multiple recessions, multiple receivers, part of a projection-receiver locking mechanism, a magnet, a clamp, a hook, a lip, a welding agent, a bonding agent, an adhesive, or combinations thereof. In other exemplary embodiments, the femoral fastening mechanism can comprise a pin inserted through the femur box securing bore **81** and the femur securing bore **87.** In such exemplary embodiments, a surgeon may use a small hammer to deform a distal end of the inserted pin in the femur securing section to thereby fixedly engage the femur fastening mechanism to the femoral component in a parasagittal plane **z** (**FIG. 11A****,** see also **FIG. 13**). In such embodiments, the femur securing bore **87** may have a maximum diameter that is greater than the maximum diameter of the femur box securing bore **81.** The increased volume of the femur securing bore **87** relative to the femur box securing bore **81** may allow the distal end of the pin to deform in the femur securing bore **87** to thereby lock the pin femur fastener in a projection-receiver locking manner. Without being bound by theory, it is contemplated that the elimination of threaded elements in the femur fastener **210** may further mitigate the possibility of premature failure resulting from any minor incidence of torsional force transfer into the femur fastener **210.**

In still other exemplary embodiments, the femoral fastening mechanism may comprise magnetic elements of opposite polarity in which a first magnetic element is disposed in the femur box **80** and the second magnetic element of opposite polarity from the first magnetic element is disposed in a transverse surface **92** that extends between the inner surfaces of the medial implant condyle **218** and the lateral implant condyle **219** of the femoral component **205.**

An exemplary rotating hinge subassembly **10** may further comprise a modular extension stop **30** that can be configured to snap fit into an extension stop portion **28** (**FIG. 3A**) of the yoke **40.** One or more bearing members **60** are pivotally connected to the head **24** of the yoke **40** (for example) via a transverse hinge pin **50** (**FIG. 4**). An end **53** of the transverse hinge pin **50** is visible in **FIG. 2B****.** A lower or inferior portion of the body **45** of the yoke **40** includes a tibial axial post **20** configured to be disposed in the tibial component **105** in a rotating relationship. The tibial axial post **20** comprises a distal end **26.** Details about these components are provided herein. It will be appreciated that in other exemplary embodiments, one bearing member **60** can be used. In still other exemplary embodiments, more than two bearing members **60** can be used.

**FIG. 2C** depicts the femoral component **205** having been secured to the distal femur 200 and the tibial component **105** having been secured to the proximal tibia **100** of the patient's knee joint using the resection and implantation techniques described above. **FIG**. **2C** shows the rotating hinge subassembly **10** just prior to insertion and attachment to the implant assembly (*i.e.,* the femoral component **205,** the meniscal insert **150,** and the tibial component **105**). As indicated, the tibial axial post **20** is inserted into the axial bore (**104,** **FIGS. 3A****,** **4**) in the tibial stem **102.** The femur box cavity **280** is sized and configured to allow the tibial axial post **20** to be inserted from a generally anterior or superior-to-inferior orientation when the femur **200** is in flexion.

**FIG. 2D** shows the rotating hinge subassembly **10** being attached to the hinge femoral component by a femur fastener **210.** As can be appreciated in **FIG. 2D****,** with the knee in flexion, the tibial axial post **20** of the yoke **40** (see also, **FIG. 4**) has been inserted into the axial bore **104** of the tibial stem **102** in a rotating relationship **R** and the femur box **80** has been seated in the femur box cavity **280.** The femur fastener **210** is inserted through a securing bore **81** in the femur box **80** and through a femur securing bore **87** (*i.e.,* a second joint element) in the femoral component **205** and in this manner secures the femur box **80** (*i.e.,* the first joint element) to the femoral component **205.** Once the femur fastener **210** secures the femur box **80** to the femoral component **205,** the first joint element is in the engaged position. That is, the first joint element (*e.g.,* the femur box **80**) is disposed in the femoral component **205** and engages the femoral component **205** in a projection-receiver locking manner.

In this manner, the rotating hinge subassembly **10** is secured to the femoral component **205** and can be said to "fixedly engage" the femoral component **205.** Other methods of selectively mechanically engaging a first joint element of the rotating hinge subassembly **10** to the femoral component **205** in a projection-receiver locking manner, a magnetic locking manner, a clamping locking manner, a bonding locking manner, an adhesive locking manner, or combinations thereof can also be said to "fixedly engage" the rotating hinge subassembly **10** to the femoral component **205.**

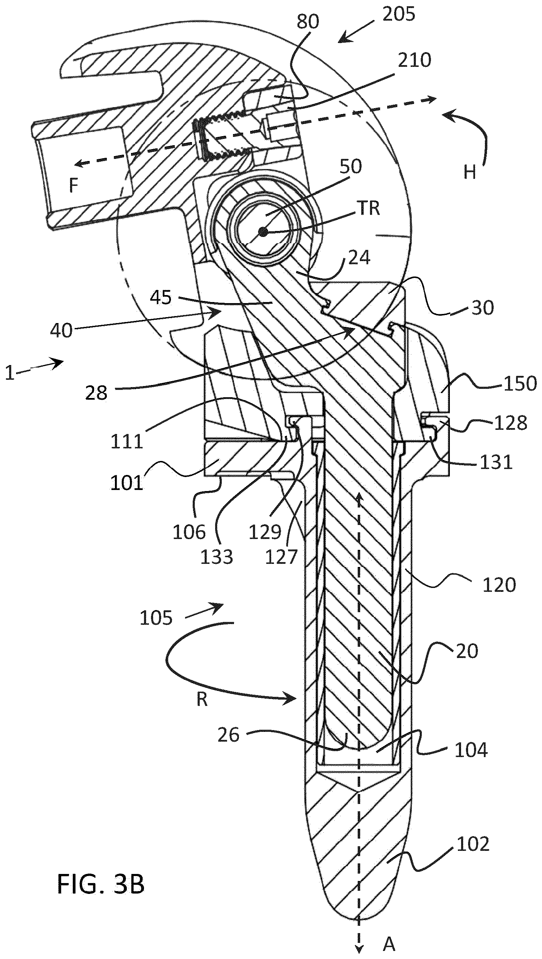

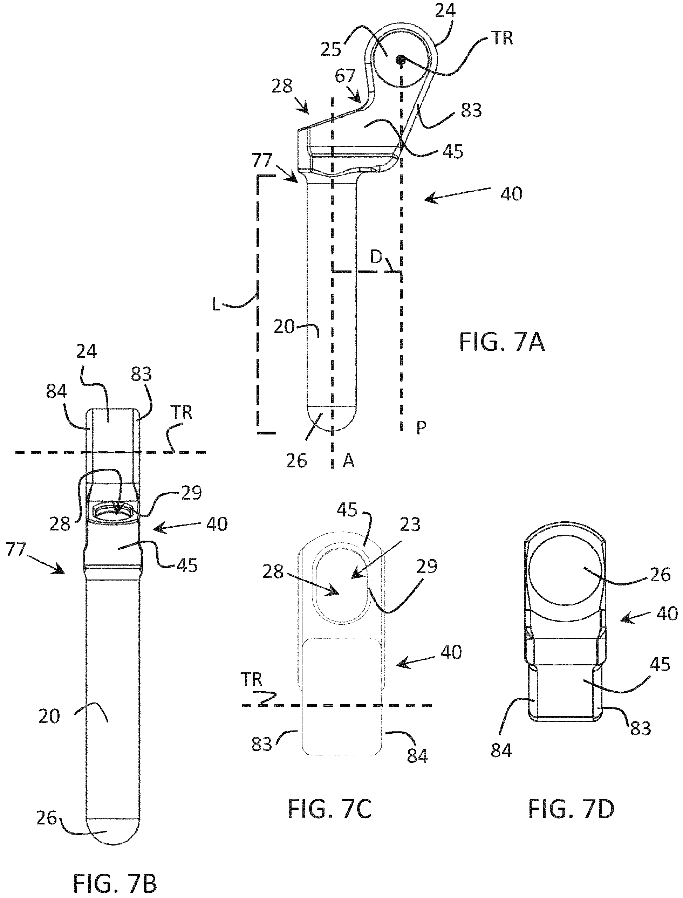

The length of the tibial axial post **20** (see **FIG. 3A**), the surrounding unresected soft tissue of the knee (*e.g.,* the medial collateral ligament "MCL" and the lateral collateral ligament "LCL"), and the position of the femoral component **205** relative to the tibial component **105** effectively secures the tibial axial post **20** in the tibial component **105** while permitting the tibial axial post **20** to rotate around a tibial axis of rotation **A** during use. In this manner, the rotating hinge subassembly **10** can be said to be disposed in a hinged and rotating configuration in the rotating hinge knee endoprosthetic implant assembly **1.** That is, the rotating hinge knee endoprosthetic implant assembly **1** is now capable of pivoting around the hinging component (*i.e*., "hingedly articulating," see also the hinge direction of rotation **H** around the transverse hinge pin **50** in **FIG. 3B**), thereby permitting the rotating hinge knee endoprosthetic implant assembly **1** to flex and extend as is readily apparent in normal use, and the femur **200** is also now capable of rotating slightly axially around a generally vertical tibial axis of rotation **A** as the rotating hinge knee endoprosthetic implant assembly **1** undergoes flexion and extension. The length **L** (**FIG. 7A**) of the tibial axial post **20** and the surrounding soft tissue prevents the yoke **40** from dislocating from the axial bore **104** of the tibial stem **102** when the knee is in full flexion. However, the distance between this distal end **26** of the tibial axial post **20** and the bottom of the tibial axial stem **102** may increase when the knee is in full flexion.

This axial rotation **R** of the femur **200** relative to the tibia **100** approximates the natural movement of a knee joint. As such the rotating hinge knee endoprosthetic implant assembly **1** avoids some of the wear forces that a fixed hinge knee would be expected to experience during normal use. Because the rotating hinge knee endoprosthetic implant assembly **1** better approximates the natural movement of a normal knee compared to a fixed hinge design, rotating hinge knees can improve patients' post-operative comfort.

In other exemplary embodiments, the tibial axial post **20** may be fixedly engaged to the inside of the tibial stem **102, or** a sleeve **120** (**FIG. 4**) so as to inhibit dislocation of the tibial axial post **20** from the axial bore **104** of the tibial stem **102.** In such embodiments, the tibial axial post **20** may comprise an expandable element that can selectively extend radially away from the tibial axial post **20** to engage the inside surface of the tibial stem **102** or the sleeve **120** (whichever is present). In still other exemplary embodiments, tibial axial post **20,** the inside of the tibial stem **102,** or the inside and outside of the sleeve **120** may have protrusions that abut an adjacent element when the tibial axial post **20** is installed inside the tibial stem **102** to fixedly engage the post member therein. In yet other exemplary embodiments, the tibial axial post **20** and the adjacent structure (*e.g.*, the sleeve **120** or the inside of the tibial stem **102**) may comprise magnets of opposite polarity.

Without being bound by theory, it is contemplated that such embodiments may be desirable in patients that suffer from ligamentous laxity. Normally, the length **L** (**FIG. 7A**) of the installed tibial axial post **20** coupled with tension from relatively nominal surrounding soft tissue (including the MCL and LCL) prevents the tibial axial post **20** from dislocating from the axial bore **104** of the tibial stem **102** when the knee is in full flexion. However, in patients that suffer from ligamentous laxity, the MCL and LCL (and other surrounding soft tissue) may not exhibit sufficient tension to prevent the dislocation of the depicted tibial axial post **20** from a depicted sleeve **120,** or inside of the tibial stem **102.** As such, fixedly engaging the tibial axial post **20** to the inside of the tibial stem **102,** or a sleeve **120** in the manner described may be desirable to prevent dislocation.

Referring back to **FIG. 2D****,** the depicted femur fastener **210** is a tapered screw. The depicted tapered screw is generally shorter than fasteners used in other rotating hinge knees. Moreover, the tapered screw having threads disclosed in the exemplary embodiment is secured to the femoral component **205,** whereas some other rotating hinge assemblies are secured with threads to the tibial component.

Without being bound by theory, it is contemplated that the significant compressive and torsional loads that the tibia **100** and tibial component **105** experience during normal use of the knee can contribute to loosening of such tibial fastening elements and can eventually compromise the future stability and effectiveness of the endoprosthesis. In designs where the tibial fastening screw is locked, normal compressive and torsional forces can eventually wear away the screw threads, thereby also loosening the rotating hinge component and compromising the effectiveness of the implant.

In embodiments in accordance with this disclosure, the condyles **218, 219** desirably transfer a majority of the femoral load (the femoral load comprising the compressive load of the body above and including the femur and torsional loads) to the condylar pads **228, 229** of the meniscal insert **150.** This is typically known as "condylar loading." The meniscal insert **150** then transfers the femoral load through the tibial component **105,** tibia **100** and ultimately the patient's foot during normal use. In this manner, the disclosed embodiments desirably avoid transferring excessive forces from the femur into the rotating hinge subassembly **10.**

However, in practice, it is contemplated that not all of the femoral loads will be transferred to the condylar pads **228, 229** of the meniscal insert **150.** Some of the compressive and torsional loads, as well as other loads such as varus, valgus, hyper-extension, flexion, and anterior and posterior drawer forces can be transferred from the femur **200** through the rotating hinge subassembly **10.** In such cases, it is contemplated that the femoral loads are transferred from the femur **200** and femoral component **205** to the femur box **80** *(i.e.,* the first joint element) and through the transverse hinge pin **50** (**FIG. 3A****)** into the head **24** and body **45** of the yoke **40** (**FIG 7A**). The portions of the yoke body **45** that are disposed within the meniscal insert **150,** then transfer these forces into the tibial component **105,** tibia **100** and ultimately the patient's foot during normal use. Without being bound by theory, it is contemplated that even in situations in which forces are transferred into the rotating hinge subassembly **10,** the transverse hinge pin **50** and yoke **40** transfer these forces through the remainder of the leg while avoiding transferring these forces to the femur fastener **210.** That is, the femoral load is transferred through the femur box **80** and possibly the transverse hinge pin **50** rather than the femur fastener **210** as described further below. By placing the femur fastener **210** in a location that permits the femur fastener **210** to mechanically engage the first joint element (*e.g*., the femur box **80**) to the femoral component **205** while removing the femur fastener **210** from the chain of force transfer, it is contemplated that embodiments in accordance with the present disclosure can avoid fastener cross threading or other signs of premature wear that would otherwise result from normal use. In certain exemplary embodiments, the location that the femur fastener **210** or other femoral fastening mechanism that mechanically engages the femur box **80** to the femoral component **205** may desirably be at a location that is co-axial with tibial rotation axis **A** when the knee is in extension (see **FIG. 3A**). Without being bound by theory, it is contemplated that having the femoral component **205** being coaxial with the tibial rotation axis **A** can further minimize torsional and other ancillary loads on the femur fastener **210** or other femoral fastening mechanism.

**FIG. 3A** provides a lateral cross-sectional extension view in which further aspects of the interconnection between the components can be visualized. The cross-section is taken along an implant-bisecting parasagittal plane (see **400,** **FIG. 13**). The knee is shown in full extension (*i.e.,* at zero degrees of flexion). In the depicted embodiment, the tibial axis of rotation **A** is substantially aligned (*i.e.,* is collinear) with the femur fastener's central axis **F** when the rotating hinge knee endoprosthetic implant assembly **1** is in extension, but not when the rotating hinge knee endoprosthetic implant assembly **1** is in flexion (see the femur fastener's central axis **F** relative to the tibial axis of rotation **A** in **FIG. 3B**). The vector of the femur fastener's central axis **F** moving toward the femoral component **205** can be representative of the engagement direction. The vector of the femur fastener's central axis **F** moving away from the femoral component **205** can be representative of a disengagement direction.

A modular extension stop **30** is provided. As discussed in more detail below, the modular extension stop **30** can be configured to snap fit into an extension stop portion **28** of the yoke **40.** Extension stops are generally used to prevent recurvatum. By making the extension stop **30** modular, it is contemplated that the surgeon can select a modular extension stop **30** that best fits the patient's need. Additionally, in some embodiments the modular extension stop **30** is omitted and an extension stop that is secured to the rotating hinge subassembly **10** in a fixed configuration can instead be provided. In such cases, the degree of potential hyperextension is limited by the patient's anatomy and by the interaction between the condyles **218, 219** of the femoral component **205** and the raised anterior surface of the meniscal insert **150.**

As shown in **FIG. 3A****,** the femur box **80** is secured to the femoral component **205** by the femur fastener **210** extending through the femur box securing bore **81.** Further, the femur box **80** and a bearing member **60** are hinged to the head **24** of yoke **40** by the transverse hinge pin **50.** In this manner, the femur **200** rotates (see **H,** **FIG. 3B**) about a single transverse axis **TR** relative to the head **24** of the yoke **40.** With the tibial axial post **20** resting in the axial bore **104** of the tibial stem **102,** the femur **200** is also free to rotate relative to the tibial component **105** about the generally midline tibial axis of rotation **A.** The degree of rotation is restricted by the patient's anatomy and by the interaction between meniscal insert **150** and the tibial component **102,** which is described further below. In the embodiment of **FIG. 3A****,** the tibial axial post **20** passes through a through-bore **108** (**FIG. 4**) formed in the meniscal insert **150** and into the axial bore **104** of the tibial stem **102.** In this manner, the rotating hinge subassembly **10** in the engaged position secures the meniscal insert **150** to the rotating hinge knee endoprosthetic implant assembly **1.** In the embodiment of **FIG. 3A****,** the modular extension stop **30** is configured to snap-fit onto the extension stop portion **28** in the body **45** of the yoke **40** in a fixed arrangement.

**FIG. 3A** further depicts the base portion **101** of the tibial component **105** further comprising an anterior hook **128** and a mid-hook **129.** Each hook **128, 129** defines a negative space between the bottom of the hooks **128, 129** and the surface of the tibial base portion **107.** The meniscal insert **150** has an anterior protrusion **131** and a mid-protrusion **133** disposed at the base **111** of the meniscal insert. The anterior protrusion **131** and a mid-protrusion **133** desirably fill the negative space defined by the bottom of the anterior hook **128** and a mid-hook **129** when the meniscal insert **150** is installed on the tibial base portion **101.** In this manner, the meniscal insert **150** is not only configured to snap-fit onto the tibial base portion, but the arrangement of the hooks **128, 129** and the protrusions **131, 133** permits and also limits rotational sliding of the meniscal insert **150** around the generally midline tibial rotational axis **A** during normal flexion and extension of the rotating hinge knee endoprosthetic implant assembly **1.**

It will be appreciated that if the surgeon elects not to dislocate the entire length of the tibial axial post **20** from the tibial stem **102,** the presence of the hinge subassembly **10** in the engaged position prevents the meniscal insert **150** from sliding out of the tibial component **105** when the knee is lifted in flexion during ambulatory movement. Other manners of securing the meniscal insert **150** to the tibial base **101** such that the meniscal insert **150** is rotatable around the generally midline axis of the tibia **A** are considered to be within the scope of this disclosure. A sleeve **120** can optionally, but desirably be disposed in the tibial stem **102** between the tibial axial post **20** and the tibial stem **102.** The sleeve **120** can be tightly fitted to the internal diameter of the tibial stem **102** and the outer diameter of the tibial axial post **20.**

**FIG. 3B** provides a side cross-section deep flexion view in which further aspects of the interconnection between the components can be visualized. The rotating hinge subassembly 10 can be configured to provide deep flexion in the range of about 100 degrees to about 138 degrees, which is ideal for a revision knee. In certain exemplary embodiments, the range may be between about 100 degrees and about 125 degrees. In deep flexion, maximum rotation of the tibial axial post **20** relative to the tibial component **105** is achieved, but is also limited by the hooks **128, 129** and the protrusions **131, 133** of the meniscal insert **150** and tibial component **102.** In addition to the pivoting motion, the femur **200** continues to hinge around the tibial axial post **20** only along the axis of the transverse hinge pin **50.**

As indicated in the side cross-sectional views of **FIG. 3A** and **3B****,** the degree of translation of the tibial axial post **20** in the axial bore **104** during flexion is determined by the configuration of the condyles **218, 219** of the femoral component **205.** If the condyles **218, 219** have a single fixed radius, the tibial axial post **20** will not translate to an appreciable degree during flexion. Likewise, if the condyles **218, 219** have a two or more radii, the tibial axial post **20** will translate upwardly in the axial bore **104** during flexion.

Aspects and features of the individual components of the rotating hinge knee endoprosthetic implant assembly **1** will now be discussed.

**FIG. 4** provides an exploded view of one exemplary embodiment of a rotating hinge knee endoprosthetic implant assembly **1.** Most of these components have been discussed above. However, additional components can be seen in the exploded view that were not clear or visible in the prior images. Reference is made to the other figures for a detailed overview of the components.

### Femur Box

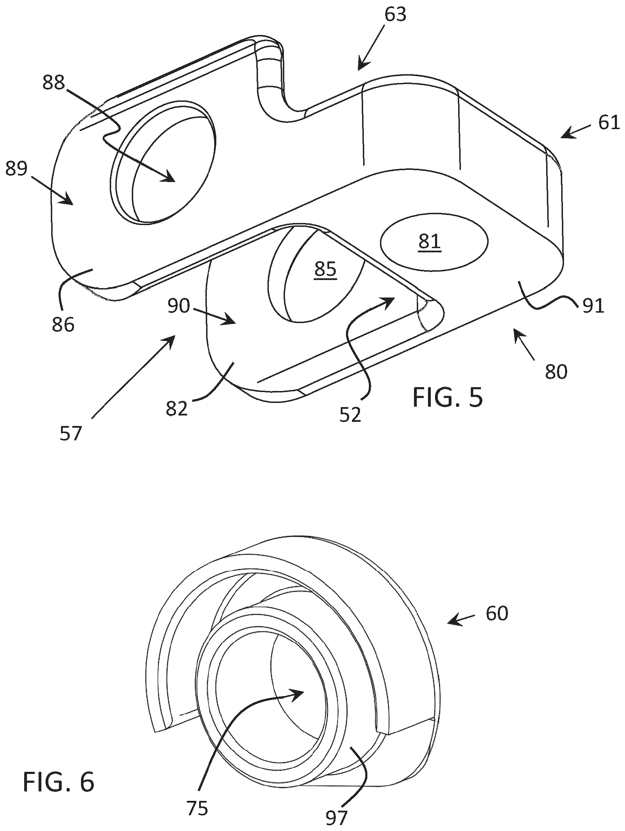

**FIG. 5** shows a perspective view of one embodiment of a femur box **80** (*i.e.,* an exemplary first joint element) for use in a rotating hinge knee endoprosthetic implant assembly 1 of the present disclosure. In the embodiment of **FIG. 5****,** the femur box **80** has a generally clevis configuration. The femur box **80** includes a main body portion **91** on anterior end **61** and a pair of opposing arms **82, 86** extending posteriorly therefrom toward a posterior end **52.** In the depicted embodiment, the arms **82, 86** take the form of clevis arms. The pair of opposing arms **82, 86** define a gap **57** between the inner surfaces **90** of the opposing arms **82, 86.** The gap **57** is sized to accommodate the width of the head **24** of the yoke **40** and desirably the width of any attached bearing members **60.** The main body portion **91** of the femur box **80** is provided with a femur box securing bore **81** extending therethrough. Additionally, the opposing arms **82, 86** are each respectively provided with arm bore **85, 88** (*i.e.,* the first arm **82** defines a first arm bore **85,** the second arm **86** defines a second arm bore **88**) extending therethrough for receiving the transverse hinge pin **50** (**FIG**. **4**). The arm bores **85, 88** are formed in the respective arms **82, 86.** The pair of arm bores **85, 88** are substantially axial in alignment with one another (along the transverse axis **TR**) to thereby provide a continuous hinge pin bore spanning the opening between the arms **82, 86.**

In certain exemplary embodiments, the femur box **80** can be made from a durable clinically proven biocompatible material that can support repeated force transfers from the femur fastener mechanism over the life of the endoprosthetic implant **1.** Example materials include cobalt chrome molybdenum alloys and titanium alloys. In other exemplary embodiments, the femur box **80** can be made from polyether ether ketone "PEEK," an organic thermoplastic polymer. PEEK is hydrophobic, which reduces any risk of the thermoplastic fusing with bone. Such properties may contribute to reduced wear of the PEEK component over time. PEEK is also radiolucent and nonmagnetic, thereby making PEEK components transparent on radiographs and compatible with magnetic imaging technologies.

In other exemplary embodiments, it is contemplated that and the femur box **80** can be made from other biocompatible, clinically proven articulating materials, including but not limited to UHMWPE and ceramic materials, including but not limited to zirconia toughened alumina ("ZTA") ceramics. It is further contemplated that if the femur box **80** is manufactured from metal, the femur box **80** may be optionally coated in zirconium oxide or niobium nitride to further reduce coefficients of friction between the articulating components and to enhance durability. In such exemplary embodiments, it is contemplated that coating the outer surface of the femur box **80,** including the outside faces of the arms **82, 86** may be desirably coated further reduce coefficients of friction. For clarity, the femur wall defining the box securing bore **81** should not be coated with a friction-reducing substance because such a substance would facilitate removal of the femur fastener **210** during normal use.

As indicated in the drawings, the femur box **80** is sized and configured to house all components of the hinge function. In embodiments, the transverse hinge pin **50** is sized to fit flush on the outside faces of the arms **82, 86.** In certain exemplary embodiments, the edges **63** of the femur box **80** are radiused for unobstructed assembly into the femur implant.

Without being bound by theory, it is contemplated that sizing the transverse hinge pin **50** to have the ends **53** of the transverse hinge pin **50,** that are disposed flush or nearly flush to the outside faces **89** of the arms **82, 86,** permits the femoral component **205** to transfer any torsional forces from the femur **200** to the femur box **80** over the combined surface areas of the outside face **89** of the arms **82, 86** and the respective ends **53** of the transverse hinge pin **50.** It will be understood that "flush" or "flushly" as used in this context means that the ends **53** of the transverse hinge pin **50** are disposed substantially coplanar with the plane that is coextensive with the outside face **89** of one or both of the arms **82, 86.**

It is contemplated that transferring any torsional forces in this manner can distribute the torsional load over a larger area thereby reducing the concentration of the torsional load in any one area. It is further contemplated that the distribution of any torsional load over a broader area can reduce torsional wear and generally prolong the useful life of the exemplary embodiments described herein. In other exemplary embodiments, one or both ends **53** of the transverse hinge pin **50** may not be disposed flush with the outside face **89** of the arms **82, 86;** rather, one or both ends **53** of the transverse hinge pin **50** may extend into the pair of arm bores **85, 88** to permit the femur box **80** to rotate around the transverse axis **TR** of the transverse hinge pin **50,** but remain within the pair of arm bores **85, 88** such that one or both ends **53** of the transverse hinge pin **50** do not extend further than the outside face **89** of the arms **82, 86.** It is contemplated that such embodiments may still provide the advantage of distributing torsional forces over a larger surface area than what was previously known.

Although the transverse hinge pin **50** is depicted as a separate element throughout the figures (see *e.g*., **FIG. 4**) it will be understood that in other exemplary embodiments, the inner sides **90** of the arms **82, 86** may comprise a portion of the transverse hinge pin **50.** In such exemplary embodiments, a medial end of the portion of the transverse hinge pin **50** extends away from the inner surface **90** of the first arm **82** into the gap **57** between the opposing arms **82, 86.** In an assembled configuration, the portion of the transverse hinge pin **50** extends into the transverse hinge bore **25** of the head **24** of the yoke **40** to thereby hingedly engage the first arm **82** of the femur box **80** to the yoke **40.** Similarly, such exemplary embodiments may further comprise a second portion of the transverse hinge pin **50** extending away from the inner surface (view obstructed in **FIG. 5****,** but see **90**) of the second arm **86** into the gap **57** between the opposing arms **82, 86.** In an assembled configuration, the second portion of the transverse hinge pin **50** extends into the transverse hinge bore **25** of the head **24** of the yoke **40** to thereby hingedly engage the second arm **86** of the femur box **80** to the yoke **40.** In such exemplary embodiments comprising this modified femur box **80,** the femur box **80** can be said to "press fit" or "interference fit" into the transverse hinge bore **25** of the yoke **40.** Furthermore, in such exemplary embodiments, the arm bores **85, 88** may be absent.

### Bearing Member

**FIG. 6** depicts an example bearing member **60** sized to have a bearing surface that extends into the transverse hinge bore **25** of the head **24** of the tibial yoke **40.** This bearing member **60** can rotate freely within the femur box **80** and around the yoke head **24** and the transverse hinge pin **50** when disposed in an installed position (see **FIG. 2**). It will be appreciated that certain exemplary embodiments can comprise more than one bearing member **60.** The bearing member **60** defines a bearing bore **75** configured to closely receive the transverse hinge pin **50** (or a portion of the transverse hinge pin **50**). In this manner, the transverse hinge pin **50** is supported by and rotates within the bearing bore **75.** The ends **53** of the transverse hinge pin **50** are disposed in the axially aligned arm bore **85, 88** of the femur box **80.** In this manner, the femur box **80** is configured to hingedly rotate around the transverse axis **TR** of the rotating hinge subassembly **10.** Other embodiments may use a bearing sleeve that circumferentially abuts the transverse hinge pin **50** and extends through the arm bores **85, 88** to achieve hinging rotation around the transverse rotational axis **TR.**

It will be understood that in other exemplary embodiments, the inner side of the bearing member **60** may comprise a portion of the transverse hinge pin **50.** In such exemplary embodiments, a medial end of the portion of the transverse hinge pin **50** extends away from the inner surface of the bearing member **60.** In an assembled configuration, the portion of the transverse hinge pin **50** extends into at least one arm bore **85** and into the transverse hinge bore **25** of the head **24** of the yoke **40** to thereby hingedly engage the bearing member **60** to the femur box **80** and the yoke **40.** Multiple bearing members **60,** each having a portion of the transverse hinge pin **50** extending from an inner surface are also contemplated. In such exemplary embodiments comprising this modified bearing member **60,** the portion of the transverse hinge pin **50** can be said to "press fit" or "interference fit" into the bearing member **60.**

In embodiments in which the bearing member **60** is configured to be disposed between a lateral side **84** of the yoke **40** and the inner surface **90** of an arm **86** of the femur box **80,** the bearing member **60** can comprise an inner hinge portion oppositely disposed from an outer hinge portion, wherein the inner hinge portion and the outer hinge portion are coaxially aligned with the transverse rotation axis **TR** in an assembled configuration. The outer hinge portion is disposed within an arm bore **88** of an arm **86** of the femur box **80** while the inner hinge portion is disposed within the transverse hinge bore **25** of the yoke **40** to thereby hingedly engage the femur box **80** to the yoke **40.** Such embodiments may further comprise a second bearing member 60 so described to hingedly engage the other arm **82** of the femur box **80** to the yoke **40.** In all such exemplary embodiments, the end **53** of the transverse hinge pin **50** desirably does not extend into the femoral component **205** in the installed configuration.

In certain exemplary embodiments, it is contemplated that the bearing member **60** can be made from a biocompatible, clinically proven articulating materials, including but not limited to cobalt chrome molybdenum alloys, titanium alloys, UHMWPE, PEEK, and ceramic materials, including but not limited to zirconia toughened alumina ("ZTA") ceramics. It is further contemplated that if the bearing member **60** is manufactured from metal, the bearing member **60** may be optionally coated in zirconium oxide or niobium nitride to further reduce coefficients of friction between the articulating components and to enhance durability. In such exemplary embodiments, it is contemplated that coating the outer surface of the bearing member **60,** may be desirably coated further reduce coefficients of friction. The bearing effectively creates a barrier between the femur box **80** and the head **24** of the yoke **40.** A zirconium oxide or niobium nitride coating may desirably substantially reduce the likelihood of metal shavings generated as the result of normal use that might otherwise arise with two metal components creating shear forces relative to one another during normal movement.

### Tibial Yoke

**FIG. 7A** shows a lateral view of one embodiment of an endoprosthetic tibial yoke **40** (*i.e.,* an exemplary second joint element) for use in a rotating hinge knee endoprosthetic implant assembly **1.** In certain exemplary embodiments, the tibial yoke **40** is a unibody *(i.e.,* a single contiguous) structure having a body member **45** and a tibial axial post **20** extending downwardly from the second end **77** of the body member **45.** The second end **77** of the body member **45** is distally disposed from a first end **67** of the body member **45.** The head **24** extends from the first end **67** of the body member **45.** The head **24** defines a transverse hinge bore **25** extending therethrough. In other exemplary embodiments, the tibial axial post **20** and body member **45** or the body member and head **24** may be separately manufactured components that are desirably assembled into the tibial yoke **40** prior to insertion into the rotating hinge knee endoprosthetic implant assembly **1.**

As seen in the lateral view of **FIG. 7A****,** the body member **45** extends away from the transverse rotational axis **TR** to provide an offset distance **D** between the transverse rotational axis **TR** of the transverse hinge bore **25** and the tibial rotation axis **A** of the tibial axial post **20.** In certain exemplary embodiments, the inner diameter of the transverse hinge bore **25** is sized to receive and articulate with an outer diameter of areas **97** of a bearing member **60** that define the bearing bore **75.** The bearing bore **75** receives the transverse hinge pin **50,** as described elsewhere herein. It will be appreciated that other ways of using a bearing member **60** to reduce the coefficient of friction between the femur box **80** and the transverse hinge pin **50** are withing the scope of this disclosure. In certain exemplary embodiments, the edges of the body member **45** are radiused.

The tibial axial post **20** further comprises a distal end **26** disposed distally from the second end **77** of the body member **45.** The distal end **26** may be chamfered or otherwise configured to ease insertion of the tibial axial post **20** into the axial bore **104** of the tibial stem **102.** In certain exemplary embodiments, the distal end **26** may comprise a conical tip. In other exemplary embodiments, the distal end **26** may be substantially hemispherical. In still other exemplary embodiments, the distal end **26** can be selected from a group of shapes including a generally convex shape, a chamfered shape, a convex conical shape, a convex hemispherical shape, and a convex frustoconical shape. In certain exemplary embodiments, the tibial axial post **20** can define a post member chamber **23,** and thereby be hollow (see **FIG. 7C****).**

**FIG. 7D** is a bottom-up view of an exemplary tibial yoke **40** depicting the distal end **26** of the tibial axial post **20,** the body **45** of the yoke **40** and the first and second lateral sides **83, 84.**

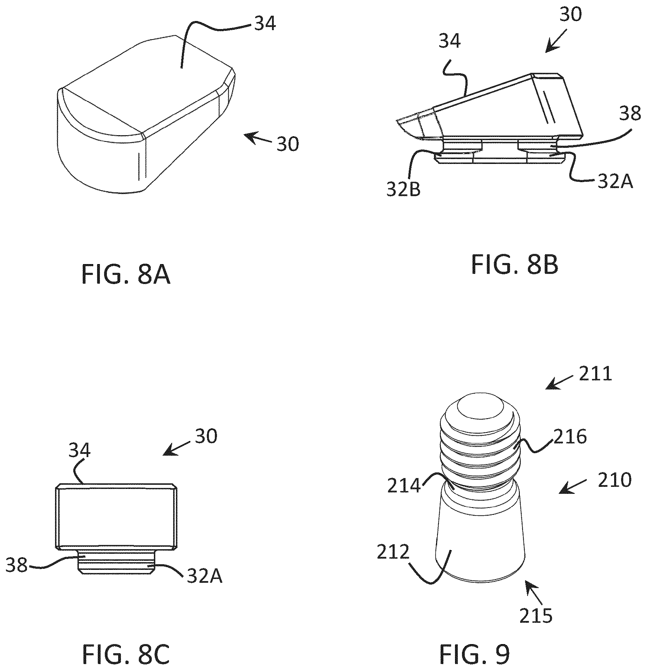

In certain exemplary embodiments, the body member **45** includes an extension stop portion **28** (**FIG. 7B, 7C**) extending on the top of the body member **45** between the head **24** and the tibial axial post **20.** As indicated in **FIG. 7A****,** in embodiments, an offset distance **D** between the midline tibial rotational axis **A** of the tibial axial post **20** and a parallel axis **P** extending orthogonally through the transverse axis **TR** of the transverse hinge bore **25** is about 12 mm to about 15 mm. The offset distance **D** defines the center of rotation of the femur **200** relative to the tibia **100.** Certain exemplary embodiments may position revision femur's center of rotation slightly more anteriorly than the native femur's center of rotation. In these exemplary embodiments, it has been discovered that allowing the femur to rotate more anteriorly can contribute to better condylar loading and force transfer away from the femur fastening mechanism. In situations in which multiple yokes **40** are provided, the multiple yokes **40** may have different size dimensions, including different offset distances **D.** The surgeon may select the appropriately sized yoke **40** with the appropriately sized offset distance **D** based on the size and the integrity of a patient's particular anatomy. The extension stop portion **28** is configured to receive and connect to a separate modular extension stop **30,** as described herein. The mechanism for connecting to the modular extension stop **30** can include snap-fit pockets **29** formed in an upper surface of the modular extension stop portion **28** for receiving matching locking tabs **32A, 32B** (**FIG. 8B**) of the modular extension stop **30.**

### Modular Extension Stop

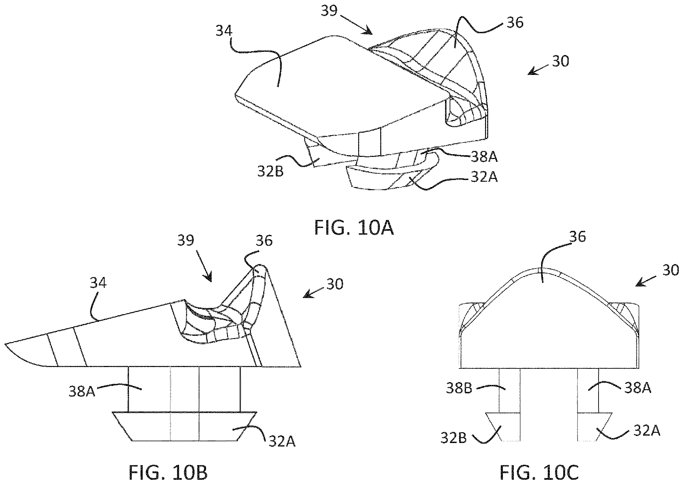

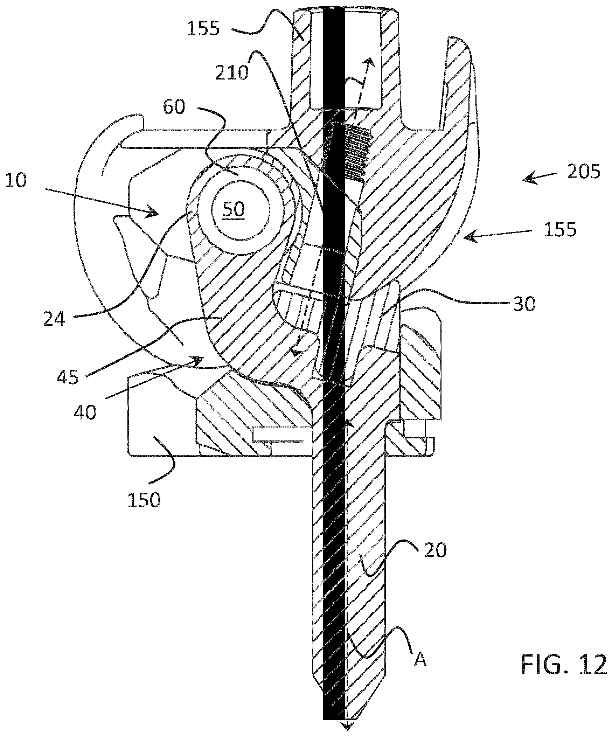

**FIGs. 8A - 8C** show views of one embodiment of an extension stop **30** for use in an exemplary rotating hinge knee endoprosthetic implant assembly **1.** The modular extension stop **30** can exist in an uninstalled position in which the modular extension stop **30** is not engaged to the rotating hinge subassembly **10.** The modular extension stop **30** can also exist in an engaged position in which the modular extension stop is engaged to the extension stop portion **28** of the rotating hinge subassembly **10.** In **FIG. 8A****,** it can be seen that the modular extension stop **30** has a substantially flat or planar top surface **34** of a posterior side. In the embodiment depicted in **FIG. 10****,** the top surface **34** rises into an abutment portion **36.** The abutment portion **36** fits within the patellar groove of the femoral component **205** when the knee is in extension. In certain exemplary embodiments, the abutment portion **36** can have a concave anterior portion that continues the patellar groove of the femoral component **205** when the knee is in extension. In this manner, an exemplary modular extension stop **30** can be configured to fit within a patellar grove of the femoral component **205** and to continue the patellar groove defined by the femoral component **205,** which permits a smooth transition between extension and flexion (see **FIG. 12**). A dip **39** separates the top surface **34** from the abutment portion **36.** This dip **39** prevents the overextension (e.g., negative flexion, or bending of the knee in the wrong direction) of the femoral component **205.**

**In** **FIG. 10C****,** an anterior view of one embodiment of a modular extension stop **30** for use in an exemplary rotating hinge knee implant assembly of the present disclosure.

The modular extension stop **30** may be made of a durable plastic material such as UHMWPE. The modular extension stop **30** can be provided in different configurations to allow for various degrees of hyperextension, such as -10°, -5°, -3°, and 0° hyperextension. In this manner, the surgeon is able to readily customize the modular extension stop **30** to the individual patient.

In embodiments, the modular extension stop **30** is configured to snap lock into an extension stop portion **28** of the tibial axial post **20.** In the embodiment shown in **FIGs. 10A, 10B,** and **10C****,** the snap locking mechanism can comprise a first snap member **32A** disposed adjacently to a second snap member **32B.** The snap members **32A, 32B** can be bendable around respective snap member stems **38A, 38B.** The snap members **32A, 32B** can fit into snap fit pockets **29** of the extension stop portion **28** of the yoke **40** when the modular extension stop **30** is in an installed position. **FIGS. 8A, 8B,** and **8C** depict alternative snap fit members **32A, 32B** that are not bendable around a snap fit stem **38.** Similar to the embodiments shown in embodiment shown in **FIGs. 10A, 10B,** and **10C** the snap members **32A, 32B of** **FIGS. 8A, 8B,** and **8C** can fit into snap fit pockets **29** of the extension stop portion **28** of the yoke **40** when the modular extension stop **30** is in an installed position. All projection-receiver locking structures that can selectively mechanically engage the modular extension stop **30** to the extension stop portion **28** of the yoke **40** are considered to be within the scope of this disclosure.

In other exemplary embodiments, the extension stop **30** is not modular, rather the extension stop **30** is integrally connected to the yoke **40** at all times. In such embodiments the extension stop **30** can be manufactured as a part of the yoke **40,** or the extension stop **30** can be permanently affixed to the extension stop portion **28** in a manner that precludes replacement of an extension stop **30** on the yoke **40** intraoperatively.

### Tapered-Head Fastener

**FIG. 9** shows a top down perspective view of one embodiment of a femur fastener **210** for use in a rotating hinge knee endoprosthetic implant assembly **1** of the present disclosure. In the pictured embodiment, the femur fastener **210** includes a threaded leading end **211,** a tapered head **212** on a trailing end **215,** and a smooth shank portion **214** between the threaded leading end **211** and the tapered head **212.** The thread **216** is typically a machined thread. The threads **216** of a threaded portion approaching the threaded leading end **211** are desirably chamfered or tapered to facilitate the installation of the femur fastener **210** into the femur box securing bore **81.** That is, the diameter of the femur fastener **210** at the threaded leading end **211** is desirably smaller than the inner diameter of the femur securing bore **87.** Guiding a smaller diameter threaded leading end **211** into a comparatively larger diameter femur securing bore **87** permits the surgeon to be less precise when initially inserting the threaded leading end **211** of the femur fastener **210** into the femur securing bore **87** and thereby is configured to facilitate engagement of the femur box **80** to the femoral component **205** via the femur fastener **210.**

To eliminate the possibility of cross-threading, surgeons can desirably start to insert the femur fastener **210** (if the femur fastener **210** has threads **216**) by first rotating the femur fastener **210** in the opposite direction from the engagement direction. This allows for the leading thread of the femur fastener **210** to eventually drop below the leading edge of the threads in the femur securing bore **87.** This may create an audible click, or the surgeon may simply register the sudden change in position. Once the surgeon registers the click or the engagement of the threads, the surgeon can then start to rotate the femur fastener **210** in the engagement direction.

As the femur fastener **210** threads into the femur securing bore **87** of the femoral component **205,** the tapered head **212** automatically locks into the femur box securing bore **81** to prevent the femur fastener **210** from backing out of the femoral component **205.** The tapered locking configuration of the femur fastener **210** enables distal assembly of the rotating hinge knee endoprosthetic implant assembly **1.** "Distal assembly" means the assembly on the femoral component **205,** which is secured to the distal portion of the patient's resected femur **200** by this point in the installation procedure. It is contemplated that by placing the knee in flexion during the subassembly installation procedure, the surgeon may have improved access to the femur box cavity **280,** especially when compared to other hinge knee endoprosthetic that require that the hinge component be secured to the tibial component **105.** The tibial component **105** can be more obstructed by the patient's soft tissue by this stage. In certain exemplary embodiments, the self-locking taper angle is eight degrees, but different tapers, such as taper angles between and including 8 to 12 degrees can be used.

The femur fastener **210** can be manufactured from any clinically proven biocompatible material. Such materials include but are not necessarily limited to cobalt chromium molybdenum alloys and titanium alloys. It will be appreciated that the femur fastener **210** can comprise any device configured to fixedly engage the femur box **80** to the femoral component **205.** Such devices may include but are not necessarily limited to pins with protruding elements, pins with negative elements configured to receive protruding elements from the femur box **80** or other interlocking assembly, bolts, rivets, clamps, interlocking teeth, interlocking hooks, and other projection-receiver locking mechanisms configured to fixedly engage the femur box **80** to the femoral component **205** such that the femur fastener **210** does not receive the load of the femur **200.**

Furthermore, and without being bound by theory, it is contemplated that the exemplary embodiments disclosed herein permit the femoral load of the femur (*i.e.,* the weight of the femur and the body above the femur due to gravity) to be primarily transferred from the femur **200** and femoral component **205** to the condylar pads **228, 229** of the meniscal insert **150.**

However, in situations where a subset of the femoral load is transferred to the rotating hinge subassembly **10,** it is contemplated that the force will be transferred from the femur **200** and femoral component **205** to the femur box **80,** transverse hinge pin **50,** and head **24** and body **45** of the yoke **40.** The bottom of the body **45** of the yoke **40** in turn transferred this load to the tibial component **105,** tibia **100,** and ultimately the patient's foot when standing or walking. The femoral load can also include the torsional load that the tibia experiences during normal ambulatory movement. The femoral load is not transferred to the femur fastener **210.** Therefore, the femur fastener **210** can be shorter than securing fasteners configured to align axially with the tibial rotational axis. In exemplary embodiments, the length of the femur fastener **210** can be about 14 mm for example. Other compatible femur fasteners **210** can have lengths between a range of about 10 mm and about 18 mm.

The transferring of the femoral load through components other than a femur fastener **210** that is configured to mechanically connect the rotating hinge subassembly **10** to the femoral component **205** may prolong the useful life of the endoprosthesis over conventional models in addition to facilitating installation of the endoprosthesis. The fact that the femur fastener **210** can be a single femur fastener **210** in some embodiments, and the fact that the femur fastener **210** can be inserted through the femur box **80** into the femoral component **205** may contribute to an overall reduction in the time required to carry out the surgical procedure.

### Femoral Component

In embodiments, the femoral component **205** is provided with features for improved joint function. A femur stem **155** is typically inserted into a femoral bore to seat the femoral component **205** on the resected femoral condyles. The femoral component **205** is configured to provide flexion greater than 120 degrees. The femoral component **205** can be configured to provide uninterrupted patellar kinematics. In certain exemplary embodiments, a femur box cavity **280** (**FIG 2A**) of about 16 to about 18 mm in length is provided for the femur box **80** of the rotating hinge subassembly **10.** The femoral component **205** can be configured to be compatible with existing revision components, such as femoral augments, offset adapters, and modular stems as well as the modular extension stop **30.**

In certain embodiments, both condyles **218, 219** can have a constant radius of curvature. Thus, a ball and socket functionality can be provided on both sides. In other embodiments, the condyles **218 , 219** can comprise multiple radii and thereby define a J-curve.

### Tibial Component

Referring to **FIG. 4****,** the tibial component **105** includes a conforming axial bore 104 for receiving the tibial axial post **20.**

The meniscal insert **150** can conform to mobile bearing features. The proximal/superior side of the tibial base portion **101** is provided with structural features to capture the meniscal insert **150** in a mobile bearing relationship, such as hook features as described further in reference to **FIGS. 3A** and **3B****.**

### Methods of Use

In operation, the rotating hinge knee endoprosthetic implant assembly **1** of the present disclosure is designed to allow for distal fixation of the rotating hinge subassembly **10** using an anterior approach.

The rotating hinge subassembly **10** can be configured to be pre-assembled, as shown in **FIG. 2B****,** rather than preassembled with the femur or tibia as in prior systems.

After the femoral component **205** and tibial component **105** are implanted, the rotating hinge subassembly **10** is inserted and rotated into place in the femoral component **205.** A single tapered-head femur fastener **210** can then be inserted to connect the hinge subassembly **10** to the femoral component **205.**

As can be seen from the foregoing descriptions, the exemplary embodiments of the invention have various advantages over prior implants and methods, including but not limited to the following.

The rotating hinge subassembly **10** is configured to be pre-assembled by itself, rather than assembled with the femoral component or tibial component as in prior systems. The transverse hinge pin **50** has a single point of insertion. After the femoral component **205** and tibial component **105** are implanted, the tibial axial post **20** of the rotating hinge subassembly **10** is inserted into the axial bore **104** of the tibial component **105.** The rotating hinge subassembly **10** can then be rotated into place to have the femur box **80** fit into femur box cavity **280** of the femoral component **205.** This configuration permits a large post length **L** of the tibial axial post **20** without the need for excessive distraction of the femoral component **205** and tibial component **105** of the rotating hinge knee endoprosthetic implant assembly **1,** which in turn permits conservation of more surrounding soft tissue. Preserving surrounding soft tissue can contribute to faster recovery times. Exemplary yokes **40** may have a post length **L** of about 40 mm to about 70 mm. Additionally, the implant configuration enables surgical methods that require only one incision to install the rotating hinged knee endoprosthetic implant **1,** since lateral placement of a hinge pin is eliminated.

Without being bound by theory, it is contemplated that certain exemplary embodiments disclosed herein, coupled with surgical practice, can constrain five of the six directions of movement of the tibial component **105** and the femoral component **205,** thereby permitting the surgeon to pre-align the tibial component **105** and the femoral component **205** along one plane of motion to facilitate installation. It will be appreciated that in practice, the femur **200** and the tibia **100** each comprise six fundamental directions of movement. That is, all movement of the femur **200** and the tibia **100** (and therefore any components disposed on the femur or tibia) can be reduced to the sum of movement along the six fundamental directions of movement.

As indicated in **FIG. 11A** (and prior to the installation of the rotating hinge subassembly **10),** the femoral component **205** and tibial component **105** can move relative to each other according to six fundamental directions of movement. That is, the femoral component **205** can move relative to the tibial component **105** (and vice versa) transversely (*i.e.,* along a transverse plane (see **198,** **FIG. 2D****)** in an anterior-posterior direction **x,** in a rotational direction **a** around the anterior-posterior axis **A-P,** in a transverse medial-lateral direction **y,** in a rotational direction **b** around the transverse medial-lateral axis **M-L,** in an upward and downward direction **z** along a parasagittal plane, and in a rotational direction **c** around the parasagittal upward-downward axis **U-D.**

An exemplary method comprises placing the tibial axial post **20** of the yoke **40** into the axial bore **104** of the tibial stem **102** while the knee is in flexion. The practice of aligning the femur box cavity **280** of the femoral component **205** with the femur box **80** effectively constrains five of the six fundamental directions of movement of the femoral component **205** relative to the tibial component **105** to the extent that the femoral component **205** is typically moveable primarily upwardly and downwardly along a parasagittal plane **z.** Any movement in the anterior-posterior direction x, or around the medial-lateral axis on the transverse plane **b** is thought to be minimal if present. Hands and instrumentation may be used to restrain the range of motion of the femur and tibial to align the femur box cavity **280** of the femoral component **205** with the femur box **80** of the rotating hinge subassembly **10.**

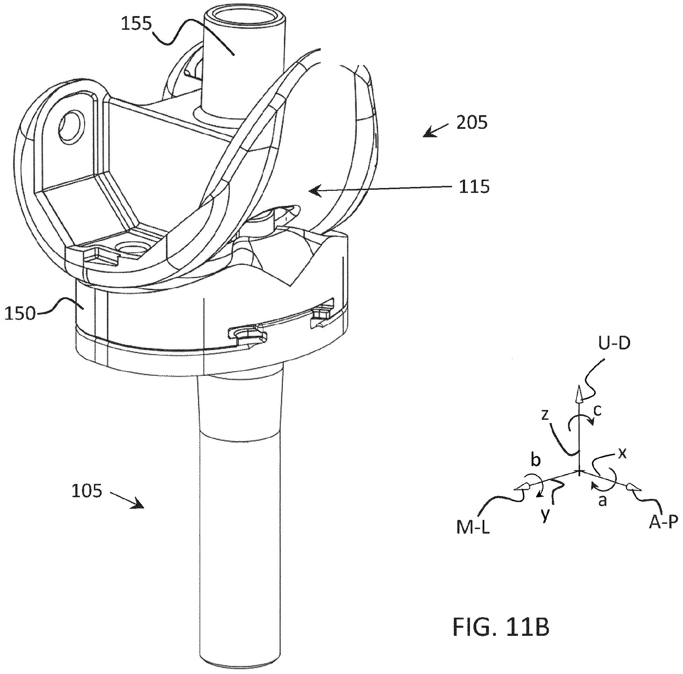

**FIG. 11B** depicts the rotating hinge subassembly **10** in the engaged position. That is, the first joint element has been disposed in the femoral component and engages the femoral component in a projection-receiver locking manner.

In embodiments, the modular extension stop **30** is configured for interaction with the femoral component **205.** The modular extension stop **30** can be provided with variable sizing and configured to snap fit into the body **45** of the yoke **40,** enabling inventory reduction. The variable sizing of the modular extension stop **30** can also be selected to correspond to specific patient anatomy *(*e.g., the modular extension stop **30** can be custom made based on preoperative images taken during a preoperative planning stage, or the modular extension stop **30** can be selected from an existing selection of modular extension stops **30** that are provided in a kit). Such modular extension stops **30** can further be selected to address specific defects in the patient's anatomy and to prevent recurvatum. The modular extension stop **30** has locking tabs **32A, 32B** extending from a bottom of the modular extension stop **30,** the locking tabs **32A, 32B** extend into the snap fit pockets **29** of the extension stop portion **28** of the yoke **40** when the modular extension stop **30** is in an installed position.

One tapered-head femur fastener **210** connects the rotating hinge subassembly **10** to the femoral component **205.** The use of a single fastener simplifies the assembly and reduces the risk of components loosening from one another during use.

The components of the rotating hinge knee endoprosthetic implant assembly **1** can be provided in the form of a surgical kit. The components of the kit are preferably arranged in a convenient format, such as in a surgical tray or case. However, the kit components do not have to be packaged or delivered together, provided that they are assembled or collected together in the operating room for use at the time of surgery. Exemplary kits may include nine rotating hinge subassemblies **10.** In certain exemplary kits, exemplary yokes **40** may have tibial axial posts **20** having one of three lengths **L.** Such exemplary yokes **40** may have a body **45** having one of three sets of dimensions. The nine rotating hinge subassemblies **10** represent the permutations and combinations of the tibial axial post length **L** and the dimensions of the body of the tibial yoke **40.** Each rotating hinge subassembly **10** may desirably have different offset distance **D** and post length **L** from the other rotating hinge subassemblies **10** in the kit.

An exemplary kit can include any suitable embodiment of a rotating hinge subassembly **10,** variations of the rotating hinge subassemblies **10** described herein, and any other rotating hinge subassemblies **10** according to an embodiment. While it is contemplated that an exemplary kit may further include one or more modular extension stops **30,** *etc.* one or more tibial components **105,** and one or more femoral components **205,** it will be appreciated that certain kits may lack some or all of these elements. Any suitable embodiment of a modular extension stop **30,** variations of the modular extension stops **30** described herein, and any other modular extension stop **30** according to an embodiment are considered to be within the scope of this disclosure. Any suitable embodiment of a tibial component **105,** variations of the tibial components **105** described herein, and any other tibial component **105** according to an embodiment are considered to be within the scope of this disclosure. Any suitable embodiment of a femoral component **205,** variations of the femoral components **205** described herein, and any other femoral component **205** according to an embodiment are considered to be within the scope of this disclosure.

Selection of a suitable number or type of rotating hinge subassembly **10,** modular extension stop **30,** tibial component **105,** and femoral component **205,** to include in a kit according to a particular embodiment can be based on various considerations, such as the procedure intended to be performed using the components included in the kit.

**FIG. 12** depicts a cross-sectional lateral view of an exemplary embodiment comprising a different modular extension stop **30.** The cross-sectional view is taken from a parasagittal plane that bisects the rotating hinge knee endoprosthetic implant assembly **1** vertically. As can be seen, at zero degrees flexion, the patella groove **115** of the femoral component **205** is sized and configured to abut against an extension stop **30.** In this manner, the rotating hinge knee endoprosthetic implant assembly 1 cannot move into hyperextension or beyond a built-in degree of hyperextension. In the exemplary embodiment of **FIG. 12****,** the femur fastening mechanism (*i.e*., the femur fastener **210** in the depicted embodiment) is non-axially aligned with the tibial axis of rotation **A** when the rotating hinge knee endoprosthetic implant assembly **1** is in full extension. That is, the femur fastener's central axis **F** is not aligned with the tibial axis of rotation **A.** It is contemplated that this non-alignment when the knee is in extension may further reduce the transfer of any torsional forces from the femur **200** into the femur fastener mechanism and thereby reduce the incidence of wear of the femur fastener mechanism during normal use.

**FIG. 13** **is** a schematic perspective view of anatomical planes relative to a person **600.** A transverse plane **198** is shown extending transversely through the person **600.** It will be appreciated that although the transverse plane **198** is shown bisecting the person horizontally at the person's midpoint, a transverse plane **198** is an imaginary plane that can be imagined to exist horizontally anywhere on the person **600** between a lateral point that is distally disposed from a medial point along the shortest possible line. In this manner, a transverse plane **198** can be said to divide a person **600** into an upper portion and a lower portion. A sagittal plane **400** is shown bisecting the person **600** vertically in an anteroposterior manner through the midpoint. It will be appreciated that although the sagittal plane **400** is shown bisecting the person, a sagittal plane **400** is an imaginary plane that can be imagined to exist vertically anywhere on the person **600** between an anterior point that is distally disposed from a posterior point along the shortest possible line. In this manner, a sagittal plane **400** divides a person into a left portion and a right portion. A sagittal plane **400** that is not disposed at the median of the person **600** is generally known as a parasagittal plane (see z , **FIG. 11A**) A coronal plane **500** is shown bisecting the person **600** vertically in a mediolateral manner through the midpoint. It will be appreciated that although the coronal plane **500** is shown bisecting the person, a coronal plane **500** is an imaginary plane that can be imagined to exist vertically anywhere on the person **600** between a lateral point that is distally disposed from a medial point along the shortest possible line. In this manner, a coronal plane **500** divides a person into a front portion and a back portion.

An exemplary knee joint endoprosthesis comprises: a tibial component, a femoral component, and a rotating hinge subassembly configured to couple to the femoral component while having a portion being configured to be disposed in the tibial component, while being rotatable around a rotational axis in the tibial component, the rotating hinge subassembly comprising: a first joint element and a second j oint element coupled with the first j oint element to be rotatable around the rotational axis, the first joint element defining: an engaged position, wherein the first joint element is disposed in the femoral component and engages the femoral component in a projection-receiver locking manner, and a disengaged position, wherein the first joint element is fully separated from the femoral component, wherein: the first joint element defines an engagement direction, wherein the first joint element is movable relative to the femoral component for transferring the first joint element from the disengaged position into the engaged position, the engagement direction runs transversely to the rotational axis, and projection and receiving elements of the projection-receiver locking manner extends non-parallelly to the engagement direction.

An exemplary rotating hinge subassembly comprises: a tibial yoke comprising: a head defining a transverse hinge bore extending through a first lateral side and a second lateral side of the head, a tibial axial post extending away from the head, and a body member disposed between the head and the tibial axial post, the body member separating the head from the tibial axial post by an offset distance; a femur box comprising: a first arm and a second arm extending posteriorly from a main body portion, the main body portion defining a femur box securing bore extending through the main body portion, wherein the first arm and the second arm respectively define axially aligned first and second bores, wherein the first arm is adjacently disposed to the first lateral side and the second arm is adjacently disposed to the second lateral side such that the first bore and the second bore axially align with the transverse hinge bore; a transverse hinge pin extending through the transverse hinge bore and into the first bore and the second bore.

An exemplary rotating hinge subassembly comprises: a tibial yoke comprising: a head defining a transverse hinge bore extending through a first lateral side and a second lateral side of the head, a tibial axial post extending away from the head, and a body member disposed between the head and the tibial axial post, the body member separating the head from the tibial axial post by an offset distance; a femur box comprising: a first arm and a second arm extending posteriorly from a main body portion, the main body portion defining a femur box securing bore extending through the main body portion, wherein the first arm and the second arm respectively define axially aligned first and second bores, wherein the first arm is adjacently disposed to the first lateral side and the second arm is adjacently disposed to the second lateral side such that the first bore and the second bore axially align with the transverse hinge bore; a transverse hinge pin extending through the transverse hinge bore and into the first bore and the second bore, wherein the transverse hinge pin does not extend beyond the arms of the femur box.

An exemplary knee joint endoprosthesis comprises: a tibial component and a femoral component, the femoral component having areas defining a femoral receiving bore; a preassembled rotating hinge subassembly configured to couple the femoral component and the tibial component to be rotatable around a rotational axis; the preassembled rotating hinge subassembly comprising: a first joint element and a second joint element coupled with the first joint element to be rotatable around the rotational axis, the first joint element having areas defining a first receiving bore, the first joint element defining: an engaged position, wherein the first joint element is disposed in the femoral component such that the first receiving bore and the femoral receiving bore align, and a fastener extends through the first receiving bore and the femoral receiving bore to thereby engage the femoral component, and a disengaged position, wherein the first joint element is fully separated from the femoral component, wherein: the first joint element defines an engagement direction, the first joint element is movable relative to the femoral component for transferring the first joint element from the disengaged position into the engaged position, the engagement direction runs transversely to the rotational axis, and the femoral bore extends in a non-parallel manner to the engagement direction.

In such an exemplary embodiment, the first joint element can be a femur box. Such an exemplary embodiment can further comprise an extension stop.

An exemplary rotating hinge knee endoprosthetic implant assembly comprises: a rotating hinge subassembly having a femur box configured to hingedly articulate around a tibial yoke via a transversely extending hinge pin, the tibial yoke comprising: a body member having a head disposed at a first end, and a tibial axial post extending from a second end of the body member, the second end of the body member being distally disposed from the first end of the body member, wherein the femur box is configured to be mechanically engaged to a femoral component via a femur fastener, wherein the femur fastener is non-axially aligned with a tibial axis of rotation when the knee is in flexion (e.g., when the rotating hinge knee endoprosthetic implant is hingedly rotating around the transverse pin at a flexion angle greater than 0 degrees) or extension (e.g., when the rotating hinge knee endoprosthetic implant is hingedly rotating around the transverse pin at a flexion angle of 0 degrees or less), and wherein the transversely extending hinge pin of the rotating hinge subassembly does not mechanically engage the femoral component in an installed configuration.

An exemplary endoprosthetic tibial yoke for a rotating hinge subassembly comprises: a body member having a head disposed at a first end of the body member; and a tibial axial post extending from a second end of the body member, the second end being distally disposed from the first end, the head defining a transverse hinge bore extending through a first lateral side and a second lateral side of the head, the transverse hinge bore defining a transverse axis extending transversely through the transverse hinge bore, the tibial axial post defining a midline axis extending longitudinally through the tibial axial post, wherein a reference axis extends substantially orthogonally through the transverse axis and substantially parallelly to the midline axis, and wherein an offset distance separates the midline axis from the reference axis.

In an exemplary embodiment of the tibial yoke, the tibial axial post, the body member, and the head are a single contiguous structure.

In an exemplary embodiment of the tibial yoke, the tibial axial post further comprises a distal end disposed distally from the second end of the body member. In such an exemplary embodiment, the distal end can have a shape selected from the group consisting essentially of a convex shape, a chamfered shape, a convex conical shape, a convex hemispherical shape, and a convex frustoconical shape.

According to the claimed embodiment of the tibial yoke, the offset distance is selected from a range of distances consisting essentially of about 12 mm to about 15 mm.

In an exemplary embodiment of the tibial yoke, the tibial axial post further comprises a post length, and wherein the post length has a value of about 40 mm to about 70 mm.

An exemplary tibial yoke may further comprise an extension stop portion extending between the head and the tibial axial post, wherein the extension stop portion comprises snap-fit pockets. In such an exemplary embodiment, the tibial yoke may further comprise a modular extension stop having locking tabs extending from a bottom of the modular extension stop, the locking tabs being configured to extend into the snap fit pockets of the extension stop portion of the yoke when the modular extension stop is in an installed position.

An exemplary rotating hinge subassembly comprises: a tibial yoke comprising: a head defining a transverse hinge bore extending through a first lateral side and a second lateral side of the head, a tibial axial post extending away from the head, and a body member disposed between the head and the tibial axial post, the body member separating the head from the tibial axial post by an offset distance; a femur box comprising: a first arm and a second arm extending posteriorly from a main body portion, the main body portion having a femoral fastening mechanism configured to engage a transverse surface of a femur box cavity of a femoral component of a knee joint endoprosthesis , wherein the first arm and the second arm respectively define axially aligned first and second arm bores, wherein the first arm is adjacently disposed to the first lateral side and the second arm is adjacently disposed to the second lateral side such that the first arm bore and the second arm bore axially align with the transverse hinge bore; a transverse hinge pin extending through the transverse hinge bore and into the first arm bore and the second arm bore.

In an exemplary embodiment of the rotating hinge subassembly the rotating hinge subassembly is preassembled.

In an exemplary embodiment of the rotating hinge subassembly the first arm and the second arm are flushly disposed against the lateral sides of the head.

In an exemplary embodiment of the rotating hinge subassembly the transverse hinge pin does not extend beyond the first and second arms of the femur box.

In an exemplary embodiment of the rotating hinge subassembly, the rotating hinge assembly may further comprise a bearing disposed between a first lateral side of the head and the first arm.

In an exemplary embodiment of the rotating hinge subassembly the femur box is made from a material from a group of materials consisting essentially of a cobalt chrome molybdenum alloy, a titanium allow, a zirconia toughened alumina ceramic, a ceramic material, an ultrahigh molecular weight polyethylene, a polyether ether ketone, combinations thereof, and other biocompatible clinically proven articulating materials.

In an exemplary embodiment of the rotating hinge subassembly the femoral fastening mechanism is selected from the group consisting essentially of a femur box securing bore configured to receive a femur fastener, a projection, a receiver, multiple projections, multiple receivers, a magnet, a clamp, a hook, a lip, a bonding agent, an adhesive, and combinations thereof.

An exemplary rotating hinge subassembly comprises: a tibial yoke comprising: a body member having a head disposed at a first end, and a tibial axial post extending from a second end of the body member, the second end being distally disposed from the first end, the head defining a transverse hinge bore extending through a first lateral side and a second lateral side of the head, the transverse hinge bore defining a transverse axis extending transversely through the transverse hinge bore, the tibial axial post defining a midline axis extending longitudinally through the tibial axial post, wherein a reference axis extends orthogonally through the transverse axis and parallelly to the midline axis, and wherein an offset distance separates the midline axis from the reference axis; a femur box comprising: a first arm extending posteriorly from a main body portion, a second arm extending posteriorly from the main body portion, the second arm opposing the first arm, the main body portion defining a femoral fastening mechanism configured to engage a transverse surface of a femur box cavity of a femoral component of a knee joint endoprosthesis, wherein the first arm defines a first bore, wherein the second arm defines a second bore, wherein the first bore axially aligns with the second bore, wherein the first arm is adjacently disposed to the first lateral side and the second arm is adjacently disposed to the second lateral side such that the first bore and the second bore axially align with the transverse hinge bore; and a transverse hinge pin extending through the transverse hinge bore and into the first bore and the second bore to thereby hingedly engage the femur box to the head of the yoke.

An exemplary knee joint endoprosthesis comprises: a tibial component; a femoral component; and a rotating hinge subassembly having a first joint element configured to be coupled to the femoral component while having a second joint element being configured to be disposed in the tibial component, while being rotatable around a rotational axis in the tibial component, the rotating hinge subassembly comprising: a first joint element and a second joint element hingedly coupled to the first joint element, the first joint element defining: an engaged position, wherein the first joint element is disposed in the femoral component and engages the femoral component in a locking manner, and a disengaged position, wherein the first joint element is fully separated from the femoral component, wherein: the first joint element defines an engagement direction, wherein the first joint element is movable relative to the femoral component for transferring the first joint element from the disengaged position into the engaged position, and the engagement direction runs sagittally to the rotational axis.

In an exemplary knee joint endoprosthesis, the locking manner is a projection-receiver locking manner and wherein projection and receiving elements of the projection-receiver locking manner are disposed coplanar with a parasagittal plane extend non-parallelly to the engagement direction. In such an exemplary knee joint endoprosthesis, the projection-receiver locking manner may comprise: the first joint element having areas defining a first receiving bore, and the femoral component having areas defining a femoral receiving bore, wherein the first receiving bore and the femoral receiving bore align, and a fastener extends through the first receiving bore and the femoral receiving bore.

In an exemplary knee joint endoprosthesis, the parasagittal plane extends through a midpoint of the femoral component.

In an exemplary knee joint endoprosthesis, the locking manner is a bonding or magnetic locking manner.

## Claims

1. A rotating hinge knee subassembly (10) comprising:
a tibial yoke (40) comprising:
a body member (45) having a head (24) disposed at a first end (67) of the body member (45);
a tibial axial post (20) extending from a second end (77) of the body member (45),
the second end (77) being distally disposed from the first end (67),
the head (24) defining a transverse hinge bore (25) extending through a first lateral side and a second lateral side of the head (24),
the transverse hinge bore (25) defining a transverse axis (TR) extending transversely through the transverse hinge bore (25),
the tibial axial post (20) defining a midline axis extending longitudinally through the tibial axial post (20),
a reference axis extends substantially orthogonally through the transverse axis (TR) and substantially parallelly to the midline axis,
an offset distance (D) separates the midline axis from the reference axis;
a femur box (80) comprising:
a first arm (82) and a second arm (86) extending posteriorly from a main body portion (91),
the main body portion (91) having a femoral fastening mechanism configured to engage a transverse surface (92) of a femur box cavity (280) of a femoral component (205) of a knee joint endoprosthesis,
wherein the first arm (82) and the second arm (86) respectively define axially aligned first and second arm bores (85, 88), the first arm (82) is adjacently disposed to the first lateral side and the second arm (86) is adjacently disposed to the second lateral side such that the first arm bore (85) and the second arm bore (88) axially align with the transverse hinge bore (25);
a transverse hinge pin (50) extending through the transverse hinge bore (25) and into the first arm bore (85) and the second arm bore (88),
wherein the first arm (82) and the second arm (86) are disposed opposite the lateral sides of the head (24), and wherein the transverse hinge pin (50) does not extend beyond the first and second arms (82, 86) of the femur box (80);
a first bearing (60) disposed between a first lateral side of the head (24) and the first arm (82); and
a second bearing (60) disposed between a second lateral side of the head (24) and the second arm (86),
**characterized in that** at least the first bearing or the second bearing is made from a polyether ether ketone,
and **in that** the offset distance (D) is selected from a range of distances of 12 mm to 15 mm.

2. The rotating hinge knee subassembly (10) of claim 1, wherein the tibial axial post (20), the body member (45), and the head (24) are a single contiguous structure.

3. The rotating hinge knee subassembly (10) according to any of claims 1 to 2, wherein the tibial axial post (20) further comprises a post length (L), and wherein the post length (L) has a value of about 40 mm to about 70 mm.

4. The rotating hinge knee subassembly (10) according to any of claims 1 to 3 further comprising an extension stop portion (28) extending between the head (24) and the tibial axial post (20), wherein the extension stop portion (28) comprises snap-fit pockets (29), and wherein the tibial yoke (40) further comprises a modular extension stop (30) having locking tabs extending from a bottom of the modular extension stop (30), the locking tabs being configured to extend into the snap fit pockets (29) of the extension stop portion (28) of the yoke (40) when the modular extension stop (30) is in an installed position.

5. The rotating hinge knee subassembly (10) according to any of claims 1 to 4, wherein the femur box (80) is made from a material from a group of materials consisting essentially of: a cobalt chrome molybdenum alloy, a titanium allow, a zirconia toughened alumina ceramic, a ceramic material, an ultrahigh molecular weight polyethylene, a polyether ether ketone, combinations thereof, and other biocompatible clinically proven articulating materials.

6. The rotating hinge knee subassembly (10) according to any of claims 1 to 5, wherein the femoral fastening mechanism is selected from the group consisting essentially of: a femur box securing bore (81) configured to receive a femur fastener (210), a projection, a receiver, multiple projections, multiple receivers, a magnet, a clamp, a hook, a lip, a bonding agent, an adhesive, and combinations thereof.

## Patentansprüche

1. Drehgelenkknieunterbaugruppe (10), umfassend:
ein tibiales Joch (40), umfassend:
ein Körperelement (45), das einen Kopf (24), der an einem ersten Ende (67) des Körperelements (45) angeordnet ist, aufweist;
eine tibiale axiale Säule (20), die sich von einem zweiten Ende (77) des Körperelements (45) erstreckt, wobei das zweite Ende (77) von dem ersten Ende (67) distal angeordnet ist,
wobei der Kopf (24) eine Quergelenkbohrung (25), die sich durch eine erste laterale Seite und eine zweite laterale Seite des Kopfs (24) erstreckt, definiert,
wobei die Quergelenkbohrung (25) eine Querachse (TR), die sich durch die Quergelenkbohrung (25) quer erstreckt, definiert,
wobei die tibiale axiale Säule (20) eine Mittellinienachse, die sich durch die tibiale axiale Säule (20) längs erstreckt, definiert,
eine Referenzachse, die sich durch die Querachse (TR) im Wesentlichen orthogonal und zu der Mittellinienachse im Wesentlichen parallel erstreckt,
ein Versatzabstand (D), der die Mittellinienachse von der Referenzachse trennt;
einen Femurkasten (80), umfassend:
einen ersten Arm (82) und einen zweiten Arm (86), die sich von einem Hauptkörperabschnitt (91) nach hinten erstrecken,
wobei der Hauptkörperabschnitt (91) einen femoralen Befestigungsmechanismus aufweist, der konfiguriert ist, um eine Queroberfläche (92) eines Femurkastenhohlraums (280) einer femoralen Komponente (205) einer Kniegelenkendoprothese in Eingriff zu nehmen,
wobei der erste Arm (82) beziehungsweise der zweite Arm (86) eine axial ausgerichtete erste und zweite Armbohrung (85, 88) definieren, der erste Arm (82) an die erste laterale Seite angrenzend angeordnet ist und der zweite Arm (86) an die zweite laterale Seite angrenzend angeordnet ist, sodass die erste Armbohrung (85) und die zweite Armbohrung (88) mit der Quergelenkbohrung (25) axial ausgerichtet sind;
einen Quergelenkstift (50), der sich durch die Quergelenkbohrung (25) und in die erste Armbohrung (85) und die zweite Armbohrung (88) erstreckt,
wobei der erste Arm (82) und der zweite Arm (86) gegenüber der lateralen Seiten des Kopfs (24) angeordnet sind und wobei sich der Quergelenkstift (50) nicht über den ersten und den zweiten Arm (82, 86) des Femurkastens (80) hinaus erstreckt;
ein erstes Lager (60), das zwischen einer ersten lateralen Seite des Kopfs (24) und dem ersten Arm (82) angeordnet ist; und
ein zweites Lager (60), das zwischen einer zweiten lateralen Seite des Kopfs (24) und dem zweiten Arm (86) angeordnet ist,
**dadurch gekennzeichnet, dass** mindestens das erste Lager oder das zweite Lager aus einem Polyetheretherketon hergestellt sind,
und **dass** der Versatzabstand (D) aus einem Abstandsbereich von 12 mm bis 15 mm ausgewählt ist.

2. Drehgelenkknieunterbaugruppe (10) nach Anspruch 1, wobei die tibiale axiale Säule (20), das Körperelement (45) und der Kopf (24) eine einzelne zusammenhängende Struktur sind.

3. Drehgelenkknieunterbaugruppe (10) nach einem der Ansprüche 1 bis 2, wobei die tibiale axiale Säule (20) ferner eine Säulenlänge (L) umfasst und wobei die Säulenlänge (L) einen Wert von etwa 40 mm bis etwa 70 mm aufweist.

4. Drehgelenkknieunterbaugruppe (10) nach einem der Ansprüche 1 bis 3, ferner umfassend einen Streckungsanschlagabschnitt (28), der sich zwischen dem Kopf (24) und der tibialen axialen Säule (20) erstreckt, wobei der Streckungsanschlagabschnitt (28) Schnappverschlusslöcher (29) umfasst und wobei das tibiale Joch (40) ferner einen modularen Streckungsanschlag (30) umfasst, der Verriegelungslaschen aufweist, die sich von einem unteren Teil des modularen Streckungsanschlags (30) erstrecken, wobei die Verriegelungslaschen konfiguriert sind, um sich in die Schnappverschlusslöcher (29) des Streckungsanschlagabschnitts (28) des Jochs (40) zu erstrecken, wenn sich der modulare Streckungsanschlag (30) in einer installierten Position befindet.

5. Drehgelenkknieunterbaugruppe (10) nach einem der Ansprüche 1 bis 4, wobei der Femurkasten (80) aus einem Material aus einer Gruppe von Materialien hergestellt ist, bestehend im Wesentlichen aus einer Cobaltchrommolybdänlegierung, einer Titanlegierung, einer zirconiumdioxidverstärkten Aluminiumoxidkeramik, einem Keramikmaterial, einem Polyethylen mit extrem hohem Molekulargewicht, einem Polyetheretherketon, Kombinationen davon und anderen biokompatiblen klinisch bewährten Artikulationsmaterialien.

6. Drehgelenkknieunterbaugruppe (10) nach einem der Ansprüche 1 bis 5, wobei der femorale Befestigungsmechanismus aus der Gruppe ausgewählt ist, bestehend im Wesentlichen aus: einer Femurkastensicherungsbohrung (81), die konfiguriert ist, um eine Femurbefestigung (210) aufzunehmen, einem Vorsprung, einer Aufnahme, mehreren Vorsprüngen, mehreren Aufnahmen, einem Magneten, einer Klemme, einem Haken, einer Lippe, einem Bindungsmittel, einem Klebstoff und Kombinationen davon.

## Revendications

1. Sous-ensemble de genou à charnière rotative (10) comprenant :
un étrier tibial (40) comprenant :
un élément de corps (45) ayant une tête (24) disposée au niveau d'une première extrémité (67) de l'élément de corps (45) ;
une tige axiale tibiale (20) s'étendant à partir d'une seconde extrémité (77) de l'élément de corps (45), la seconde extrémité (77) étant disposée distalement par rapport à la première extrémité (67),
la tête (24) définissant un alésage de charnière transversal (25) s'étendant à travers un premier côté latéral et un second côté latéral de la tête (24),
l'alésage de charnière transversal (25) définissant un axe transversal (TR) s'étendant transversalement à travers l'alésage de charnière transversal (25),
la tige axiale tibiale (20) définissant un axe de ligne médiane s'étendant longitudinalement à travers la tige axiale tibiale (20),
un axe de référence s'étend de façon sensiblement orthogonale à travers l'axe transversal (TR) et de façon sensiblement parallèle à l'axe de ligne médiane,
une distance de décalage (D) sépare l'axe de ligne médiane de l'axe de référence ;
une boîte fémorale (80) comprenant :
un premier bras (82) et un second bras (86) s'étendant postérieurement à partir d'une partie de corps principale (91),
la partie de corps principale (91) ayant un mécanisme de fixation fémoral conçu pour venir en prise avec une surface transversale (92) d'une cavité de boîte fémorale (280) d'un composant fémoral (205) d'une endoprothèse d'articulation de genou,
dans lequel le premier bras (82) et le second bras (86) définissent respectivement des alésages de premier et second bras (85, 88) alignés axialement, le premier bras (82) est disposé de manière adjacente au premier côté latéral et le second bras (86) est disposé de manière adjacente au second côté latéral de telle sorte que l'alésage de premier bras (85) et l'alésage de second bras (88) s'alignent axialement sur l'alésage de charnière transversal (25) ;
une broche de charnière transversale (50) s'étendant à travers l'alésage de charnière transversal (25) et dans l'alésage de premier bras (85) et l'alésage de second bras (88),
dans lequel le premier bras (82) et le second bras (86) sont disposés à l'opposé des côtés latéraux de la tête (24) et dans lequel la broche de charnière transversale (50) ne s'étend pas au-delà des premier et second bras (82, 86) de la boîte fémorale (80) ;
un premier palier (60) disposé entre un premier côté latéral de la tête (24) et le premier bras (82) ; et
un second palier (60) disposé entre un second côté latéral de la tête (24) et le second bras (86),
**caractérisé en ce qu'**au moins le premier palier ou le second palier est constitué d'un polyéther éther cétone,
et **en ce que** la distance de décalage (D) est choisie dans une plage de distances allant de 12 mm à 15 mm.

2. Sous-ensemble de genou à charnière rotative (10) selon la revendication 1, dans lequel la tige axiale tibiale (20), l'élément de corps (45) et la tête (24) sont une unique structure contiguë.

3. Sous-ensemble de genou à charnière rotative (10) selon l'une quelconque des revendications 1 à 2, dans lequel la tige axiale tibiale (20) comprend en outre une longueur de tige (L), et dans lequel la longueur de tige (L) a une valeur d'environ 40 mm à environ 70 mm.

4. Sous-ensemble de genou à charnière rotative (10) selon l'une quelconque des revendications 1 à 3 comprenant en outre une partie de butée d'extension (28) s'étendant entre la tête (24) et le montant axial tibial (20), dans lequel la partie de butée d'extension (28) comprend des poches d'encliquetage (29), et dans lequel l'étrier tibial (40) comprend en outre une butée d'extension modulaire (30) ayant des languettes de verrouillage s'étendant à partir d'un fond de la butée d'extension modulaire (30), les languettes de verrouillage étant conçues pour s'étendre dans les poches d'encliquetage (29) de la partie de butée d'extension (28) de l'étrier (40) lorsque la butée d'extension modulaire (30) est dans une position installée.

5. Sous-ensemble de genou à charnière rotative (10) selon l'une quelconque des revendications 1 à 4, dans lequel la boîte fémorale (80) est constituée d'un matériau parmi un groupe de matériaux constitué essentiellement d'un alliage de cobalt-chrome-molybdène, d'un alliage de titane, d'une céramique d'alumine renforcée à la zircone, d'un matériau céramique, d'un polyéthylène de masse moléculaire ultra-élevée, d'une polyéther-éther-cétone, des combinaisons de ceux-ci et d'autres matériaux d'articulation biocompatibles et cliniquement éprouvés.

6. Sous-ensemble de genou à charnière rotative (10) selon l'une quelconque des revendications 1 à 5, dans lequel le mécanisme de fixation fémoral est choisi dans le groupe constitué essentiellement : d'un alésage de fixation de boîte fémorale (81) conçu pour recevoir un élément de fixation fémoral (210), d'une saillie, d'un récepteur, de multiples saillies, de multiples récepteurs, d'un aimant, d'une pince, d'un crochet, d'une lèvre, d'un agent de liaison, d'un adhésif, et des combinaisons de ceux-ci.
